# EUROPEAN PATENT SPECIFICATION

(11) **EP 4 558 042 B1**
(45) Date of publication and mention of the grant of the patent: **09.12.2026**
(21) Application number: 23744775.0
(22) Date of filing: 20.07.2023
(51) Int. Cl.: A61B 5/107, A61B 5/318

(54) **METHODS TO DETERMINE THE MORPHOLOGY AND THE LOCATION OF A HEART WITHIN A TORSO**
VERFAHREN ZUM BESTIMMEN DER MORPHOLOGIE UND DER LAGE EINES HERZENS INNERHALB EINES OBERKÖRPERS
PROCÉDÉS POUR DÉTERMINER LA MORPHOLOGIE ET L'EMPLACEMENT D'UN COEUR DANS UN TORSE

(30) Priority: 20.07.2022 EP 22382699
(43) Date of publication of application: 28.05.2025
(73) Proprietor: Corify Care, S.L., 28009 Madrid (ES)
(72) Inventor: MILAGRO SERRANO, Javier, 28009 Madrid (ES); MARTÍNEZ CLIMENT, Andreu, 28009 Madrid (ES); HERNÁNDEZ ROMERO, Ismael, 46022 Valencia (ES); GUILLEM SÁNCHEZ, María, 46022 Valencia (ES)
(74) Representative: ABG Intellectual Property Law, S.L.
(86) International application number: PCT/EP2023/070170
(87) International publication number: WO 2024/018009

(56) References cited:
- WO-A1-2017/035522
- CN-A- 105 796 094
- COLL-FONT JAUME ET AL: "ECG-based reconstruction of heart position and orientation with Bayesian optimization", 2017 COMPUTING IN CARDIOLOGY (CINC), CCAL, 24 September 2017 (2017-09-24), pages 1 - 4, XP033343563, DOI: 10.22489/CINC.2017.054-387

## Description

### TECHNICAL FIELD OF THE INVENTION

The present invention is framed within the field of non-invasive estimation of epicardial electrical activity. The present invention defines a method for the automatic and non-invasive determination of the location of at least one portion of a subject's heart within the subject's torso. The present invention also defines a method for obtaining a three-dimensional model of at least one portion of a subject's heart.

### BACKGROUND OF THE INVENTION

Cardiac arrhythmias are an ensemble of pathologies in which the rhythmic contraction of the heart, or of some of its chambers, becomes irregular. Mechanical contraction of the heart is preceded by electrical activation, which starts in the sinus node and propagates to the whole cardiac tissue in each beat. However, during an arrhythmia, this electrical propagation is not regular, thus leading to an inefficient mechanical contraction and hence to an inadequate blood pump. Therefore, cardiac arrhythmias can cause inadequate oxygenation of the tissues and organs of the body, which in the more severe cases can turn lethal.

The clinical management of arrhythmias requires a proper understanding of the mechanisms originating and sustaining them. The gold standard for arrhythmia diagnosis is the standard 12-lead electrocardiogram (ECG). By placing a reduced number of surface electrodes on a subject's torso, the analysis of ECG traces allows to assess cardiac electrical activity and the presence and type of arrhythmia, since each type of arrhythmia produces particular patterns in the ECG traces that can be interpreted by a cardiologist.

Despite the ECG plays a fundamental role in the diagnosis of arrhythmia, its importance for treatment is limited. The most effective treatment for cardiac arrhythmia is cardiac ablation, consisting in burning the specific regions of the cardiac tissue responsible of causing and/or maintaining the arrhythmia, using invasive catheters which deliver heat, radiofrequency energy or cold to the target tissues. In this context, ECG traces are not enough to determine said target regions, since they do not allow to assess electrical propagation on the cardiac tissue, neither the status of cardiac substrate. For this purpose, invasive cardiac mapping systems are required. These systems generate electro-anatomic maps that allow to visualize both electrical activity propagation and the electrical status of cardiac substrate. Said electro-anatomic maps are constructed from the acquisition of intracavitary electrograms (EGMs) recorded by the use of invasive catheters, which are placed within the heart of the patient under study.

Although invasive cardiac mapping systems provide much larger information than the ECG for the identification of the mechanisms underlying arrhythmia, they are not exempt of limitations: they do not allow to map the whole cardiac substrate during a single beat, and they are expensive, time-consuming and require an invasive procedure, with associated risks. Moreover, invasive cardiac mapping can only be performed during an electrophysiological intervention in a dedicated electrophysiology room. Thus, these systems are not useful to decide which patients are the best candidates for ablation, or to plan ablation interventions in advance.

To overcome the limitations of invasive cardiac mapping systems, non-invasive cardiac mapping systems were developed. Non-invasive cardiac mapping systems are based on ECG imaging (ECGI), which allows to estimate the electrical activity in the epicardium of the heart using only body surface potentials information, and a geometrical model of the subject's torso and heart. ECGI-based systems allow to visualize electro-anatomic maps representing cardiac electrical activity in the whole heart, or in the cardiac chambers of interest, during a single heartbeat, and requiring a much lower amount of time in comparison with invasive cardiac mapping systems. Additionally, the direct risks associated with the use of non-invasive cardiac mapping systems are reduced, being similar to that of the use of surface electrodes.

ECGI-based systems usually require a computerized tomography scan or another medical image modality of the whole torso of the subject under study, in order to obtain a three-dimensional geometric model of the subject's torso and heart and, importantly, of the precise location of the heart within the torso. In addition, in some cases the subject must have the surface electrodes already placed during this process, ensuring that the position of the surface electrodes does not change after the medical images acquisition. Examples of systems requiring this process are disclosed in US 20150335259 A1 and US 20160331263 A1. However, the requirement of medical imaging of the whole torso and with the measurement electrodes already placed in their final position limits the clinical applications of non-invasive cardiac mapping systems. On one hand, the use of medical imaging is not justified in the context of an electrophysiological study in all the cases and hospitals, and in those in which it is, it implies the interaction between different clinical units, thus complicating patients' management and records. On the other hand, the use of ECGI-based systems is also prevented during regular consultation or in order to decide the best treatment strategy for each patient and to plan an ablation intervention in advance.

Whereas a geometric model of the torso can be obtained using conventional image acquisition techniques, it is not possible to obtain an accurate model of the heart of a particular subject without more specific imaging modalities. For this reason, one possibility lies on the use of a database of previously obtained cardiac geometries, so that the best-matched model is selected. For example, in US 2012283587 A1 and WO 2019093877 A2 a database with reference cardiac models is employed. Selection of the cardiac model can be based on a variety of criteria, such as selecting the heart of a subject with a similar torso, or that of a patient with similar pathologic conditions, thus relying on an approximation. Furthermore, the use of such approaches requires to locate the cardiac geometry in its correct position within the torso geometry, which is not trivial if the relative position of both geometries is unknown a priori. The precision in the location of the heart is of paramount importance for the proper estimation of epicardial electrical activity, and errors in the estimation of said location directly influence the results of the non-invasive characterization of cardiac activity.

Some previous documents have described means for estimating heart position or variations of such. For example, in WO 2019093877 A2, a method for locating the heart of a patient is described. Nevertheless, the method requires the identification of certain anatomic structures on the subject's torso, such as the sternum and the xiphoid, whose relationship with the heart's position can vary among subjects.

Additionally, in WO 2017035522 A1, a method for assessing changes in the position of the heart using electrical information is presented. However, the described methodology is not intended for locating the absolute position of the heart inside a subject's torso.

Other relevant prior art is disclosed in CN105796094.

### SUMMARY OF THE INVENTION

The present invention provides a method for determining the location of at least one portion of a subject's heart. The invention also provides a method for estimating the morphology of at least one portion of a subject's heart and a method for determining at least one region of interest within the cardiac tissue. In the dependent claims, embodiments of the invention are defined.

In a first inventive aspect, the invention provides a computer-implemented method for determining the location of at least one portion of a subject's heart within the subject's torso, the method comprising the following steps:
(a) providing:
   - a three-dimensional torso model of the subject, the torso model being defined by a plurality of vertices and a plurality of faces, each of said faces determined by at least three vertices and at least three edges, each edge connecting a pair of vertices,
   - the position of each sensor of a plurality of sensors placed on the subject's torso, and
   - a body surface potential measured by each sensor;
(b) interpolating the body surface potential in each vertex of the torso model based on the body surface potentials measured by the sensors;
(c) defining a plurality of electrical axes, each electrical axis being an imaginary straight line connecting a vertex with another vertex, the body surface potential of which has the greatest morphological similarity but inverse polarity;
(d) determining the location of a point of electrical symmetry in the torso model based on the plurality of electrical axes; and
(e) determining the location of the geometrical center of the at least one portion of the subject's heart in the torso model based on the determined point of electrical symmetry.

The present invention provides a method for the automatic determination of the location of at least one portion of a subject's heart within the subject's torso. The at least one portion of the subject's heart can be the whole heart, or a part thereof, such as the upper cardiac chambers or the lower cardiac chambers. The term "subject" refers to any mammalian subject, particularly a human subject, whose heart is located within their torso.

According to the present invention input data is provided, the input data comprising or consisting of:
- a three-dimensional torso model of the subject,
- the position of each sensor of a plurality of sensors placed on the subject's torso, and
- a body surface potential measured by each sensor.

In the context of the present invention, a three-dimensional torso model is a convex hull comprising a plurality of vertices and a plurality of faces, each of said faces determined by at least three vertices and at least three edges, each edge connecting a pair of vertices. Particularly, each of said faces is determined by three vertices and three edges connecting each pair of vertices.

The number of sensors used for measuring the body surface potentials can be lower than the total number of vertices in the torso model. If a body surface potential has been measured at the location of a vertex, the measured body surface potential is taken to correspond to the body surface potential at said vertex. For other vertices, body surface potentials are determined by interpolating at the position of the vertices the measured body surface potentials. As a result of step (b) of the method, a body surface potential at each vertex of the torso model is obtained.

Based on the body surface potentials, a plurality of electrical axes is defined in step (c) of the method. Each electrical axis is an imaginary straight line connecting a vertex with another vertex, wherein the body surface potentials of connected vertices have the greatest morphological similarity but inverse polarity.

Throughout the whole document, the morphological similarity between two body surface potentials will be understood as a measure or assessment of the similarity between them based on their morphological or shape characteristics. It involves comparing the shapes, structures, and/or patterns of the body surface potentials rather than their overall magnitude or frequency content.

Based on the plurality of defined electrical axes, the location of a point of electrical symmetry in the torso model is determined in step (d). Based on the location of the point of electrical symmetry, the location of the geometrical center of the at least one portion of the subject's heart in the torso model is determined in step (e).

The terms "point of electrical symmetry" and "electrical center" are herein used interchangeably. The terms "geometrical center" and "O point" are herein used interchangeably to refer to the point within the torso model at which the geometrical center of the heart and/or of a portion thereof (such as the upper and/or lower chambers) is located.

Advantageously, the application of the method of the present invention is not limited to cardiac arrhythmia patients or to electrophysiological interventions but can be also used in healthy subjects and/or during regular consultation or in other non-clinical scenarios. Also, even when the body surface potentials are measured during occurrence of an arrhythmia, the method of the present invention provides an accurate determination of the location of the at least one portion of the subject's heart within the torso model.

The present invention is advantageous with respect to state-of-the-art approaches in that the position of the at least one portion of the heart can be located using only information of the subject's torso geometry, sensor position and body surface potential (i.e. surface cardiac electrical activity). In difference with previous approaches, no a priori information of the cardiac geometry is required in order to determine the location of such within the torso. Also, the present invention allows determining the location of the at least one portion of the subject's heart without requiring the identification of particular anatomic structures of the subject.

The determination of the location of the heart or of a portion thereof is relevant for the proper non-invasive characterization of epicardial electrical activity, which in turn has application on several scenarios beyond the analysis of cardiac arrhythmia, such as the screening of the cardiac electrical function in the general population. Additionally, the presented methodology can be also relevant in non-clinical scenarios which require an evaluation of cardiac electrical activity, such as in sport sciences.

Furthermore, in addition to the geometric center of the at least one portion of the heart, the location of the point of electrical symmetry of the at least one portion of the heart within the torso is determined, which has the application of e.g. determining a personalized coordinates reference system for the cardiac electrical activity in a subject.

In an embodiment, the step (c) of the method comprises, for each vertex:
- determining the morphological similarity between the body surface potential in said vertex and the body surface potential in each of the other vertices of the torso model;
- comparing the determined morphological similarities; and
- defining the electrical axis between said vertex and the vertex for which the morphological similarity is the highest and whose body surface potential has inverse polarity.

In this embodiment, for a specific vertex of the torso model, the morphological similarities between the body surface potential in said vertex and the body surface potentials in the rest of the vertices of the torso model are determined and compared to establish which of such vertices has the body surface potential which has inverse polarity and is the most morphologically similar to the body surface potential of the specific vertex. After this, an electrical axis is defined between the two vertices.

The plurality of electrical axes of step (c) is defined by repeating the previous steps for all the vertices of the torso model.

In an embodiment, the comparison of the morphological similarities comprises sorting them from the lowest to the highest and the selection of the vertex for which the morphological similarity is the highest. In an embodiment, the comparison of the morphological similarities comprises sorting them from the highest to the lowest and the selection of the vertex for which the morphological similarity is the highest.

In an embodiment, the morphological similarity between body surface potentials may be determined in different ways, such as by correlation, least squares, dynamic time warping, covariance, vector distance or others.

In an embodiment, the step (c) of the method comprises determining, for each vertex:
- the correlation of the body surface potential in said vertex with the body surface potential in each of the other vertices and defining the electrical axis between said vertex and the vertex having the greatest negative correlation; or
- the least squares value of the body surface potential in said vertex with the body surface potential in each of the other vertices and defining the electrical axis between said vertex and the vertex having the lowest least squares value but inverse polarity; or
- the dynamic time warping deformation of the body surface potential in said vertex with the body surface potential in each of the other vertices and defining the electrical axis between said vertex and the vertex having the lowest dynamic time warping deformation but inverse polarity; or
- the covariance of the body surface potential in said vertex with the body surface potential in each of the other vertices and defining the electrical axis between said vertex and the vertex having the greatest negative covariance; or
- the vector distance of the body surface potential in said vertex with the body surface potential in each of the other vertices and defining the electrical axis between said vertex and the vertex having the lowest vector distance but inverse polarity.

In this embodiment, different methodologies are defined to determine the morphological similarity between the body surface potentials of the vertices.

In a particular embodiment, the morphological similarity is determined by calculating the correlation of the body surface potential in a specific vertex with the body surface potential in each of the other vertices of the torso model. In this particular embodiment, an electrical axis is defined between said specific vertex and the vertex of the torso model for which the greatest negative correlation has been calculated.

The plurality of electrical axes of step (c) is defined by repeating the previous steps for all the vertices of the torso model.

In an embodiment, an electrical axis is defined between two vertices only if the correlation between their body surface potentials reaches a predefined threshold. According to this embodiment, the vertices having a body surface potential which does not comply with a minimum correlation threshold with the body surface potential of any other vertex are kept disconnected, such that no electrical axis is defined for them.

In a particular embodiment, the morphological similarity is determined by calculating the least squares value of the body surface potential in a specific vertex with the body surface potential in each of the other vertices of the torso model. In this particular embodiment, an electrical axis is defined between said specific vertex and the vertex of the torso model for which the lowest least squares value has been calculated and whose body surface potential has inverse polarity (compared with the polarity of the body surface potential of the specific vertex).

The plurality of electrical axes of step (c) is defined by repeating the previous steps for all the vertices of the torso model.

In an embodiment, an electrical axis is defined between two vertices only if the least squares value between their body surface potentials is below a predefined threshold. According to this embodiment, the vertices having a body surface potential which does not comply with a maximum least squares threshold with the body surface potential of any other vertex are kept disconnected, such that no electrical axis is defined for them.

In a particular embodiment, the morphological similarity is determined by calculating the dynamic time warping deformation of the body surface potential in a specific vertex with the body surface potential in each of the other vertices of the torso model. In this particular embodiment, an electrical axis is defined between said vertex and the vertex of the torso model for which the lowest dynamic time warping deformation has been calculated and whose body surface potential has inverse polarity (compared with the polarity of the body surface potential of the specific vertex).

The plurality of electrical axes of step (c) is defined by repeating the previous steps for all the vertices of the torso model.

In an embodiment, an electrical axis is defined between two vertices only if the dynamic time warping deformation between their body surface potentials is below a predefined threshold. According to this embodiment, the vertices having a body surface potential which does not comply with a maximum deformation threshold with the body surface potential of any other vertex are kept disconnected, such that no electrical axis is defined for them.

In a particular embodiment, the morphological similarity is determined by calculating the covariance of the body surface potential in a specific vertex with the body surface potential in each of the other vertices of the torso model. In this particular embodiment, an electrical axis is defined between said specific vertex and the vertex of the torso model for which the greatest negative covariance has been calculated.

The plurality of electrical axes of step (c) is defined by repeating the previous steps for all the vertices of the torso model.

In an embodiment, an electrical axis is defined between two vertices only if the covariance between their body surface potentials reaches a predefined threshold. According to this embodiment, the vertices having a body surface potential which does not comply with a minimum covariance threshold with the body surface potential of any other vertex are kept disconnected, such that no electrical axis is defined for them.

In a particular embodiment, the morphological similarity is determined by calculating the vector distance of the body surface potential in a specific vertex with the body surface potential in each of the other vertices of the torso model. In this particular embodiment, an electrical axis is defined between said specific vertex and the vertex of the torso model for which the lowest vector distance has been calculated and whose body surface potential has inverse polarity (compared with the polarity of the body surface potential of the specific vertex).

The plurality of electrical axes of step (c) is defined by repeating the previous steps for all the vertices of the torso model.

In an embodiment, an electrical axis is defined between two vertices only if the vector distance between their body surface potentials is below a predefined threshold. According to this embodiment, the vertices having a body surface potential which does not comply with a maximum distance threshold with the body surface potential of any other vertex are kept disconnected, such that no electrical axis is defined for them.

In an embodiment, in step (d) the location of a point of electrical symmetry in the torso model based on the plurality of electrical axes is determined:
- as the intersection of the plurality of electrical axes; or
- within a region determined by the plurality of electrical axes.

In an embodiment, in step (e) the location of the geometrical center of the at least one portion of the subject's heart in the torso model is determined:
- by making coincident said geometrical center with the point of electrical symmetry; or
- by applying an offset correction to the location of the point of electrical symmetry.

In an embodiment, the offset correction is estimated from one or several particular characteristics of the subject (such as physical and/or electrical characteristics) and/or from a population of subjects with known geometrical and electrical centers of the at least one portion of the heart.

In an embodiment, the torso model is obtained by one of the following options:
- by automatic, semi-automatic, or manual segmentation of images obtained using an imaging system, wherein images are particularly images of the subject;
- from a database of previously generated torso models; or
- generating a torso model from at least one mathematical model representing different subject characteristics.

In a particular embodiment, the torso model is obtained by automatic, semi-automatic, or manual segmentation of images obtained using an imaging system. Particularly, the torso model is obtained by automatic segmentation of images using an imaging system.

In an embodiment, the torso model is obtained by automatic, semi-automatic, or manual segmentation of images obtained using an imaging system, wherein:
- the images are medical images and the imaging system is a medical imaging system, such as magnetic resonance or computerized tomography scan; or
- the images are non-medical images and the imaging system is a non-medical imaging system, such as photogrammetry, video recordings or speckle interferometry.

In a particular embodiment, the torso model is obtained by automatic, semi-automatic, or manual segmentation of images, wherein the images are non-medical images and the imaging system is a non-medical imaging system, such as photogrammetry, video recordings or speckle interferometry. Particularly, images are at least two 2D images obtained using a conventional camera.

In an embodiment, the torso model is obtained by automatic segmentation of images obtained using a conventional camera.

In an embodiment, the position of each sensor on the subject's torso is determined using medical or non-medical imaging analysis techniques.

In an embodiment, the position of each sensor on the subject's torso is determined by one or several from the following options:
- automatic, semi-automatic, or manual segmentation of images obtained using an imaging system, wherein images are particularly images of the subject; or
- using an artificial intelligence-based approach for the identification of the sensor position based on the detection of readable codes, labels and/or drawings provided on the sensors.

In an embodiment the position of each sensor on the subject's torso is determined by automatic, semi-automatic, or manual segmentation of images obtained using an imaging system, wherein:
- the images are medical images and the imaging system is a medical imaging system, such as magnetic resonance or computerized tomography scan; or
- the images are non-medical images and the imaging system is a non-medical imaging system, such as photogrammetry or video recordings.

In an embodiment, the position of each sensor on the subject's torso is determined by automatic segmentation of images using an imaging system.

In an embodiment, the position of each sensor on the subject's torso is determined by automatic segmentation of images, wherein the images are non-medical images and the imaging system is a non-medical imaging system, such as photogrammetry or video recordings. Particularly, images are at least two 2D images obtained using a conventional camera.

In an embodiment, the position of the sensors on the subject's torso is obtained by automatic segmentation of images obtained using a conventional camera.

In an embodiment, the torso model and/or the position of each sensor on the subject's torso is obtained by automatic segmentation of images obtained using a conventional camera.

In an embodiment, the plurality of sensors placed on the subject's torso comprise from 100 to 150 electrodes. Particularly, the plurality of sensors placed on the subject's torso comprise 128 electrodes.

In an embodiment, the sensors are homogeneously distributed over the whole torso surface.

In an embodiment, the plurality of sensors is arranged in a sensor vest configured for the acquisition of body surface potentials.

In an embodiment, in step (b) the interpolation technique is Laplacian interpolation, Radial Basis Function interpolation, Cubic Splines interpolation and/or Nonmanifold Laplacian interpolation. In an embodiment, the interpolation technique is Nonmanifold Laplacian interpolation.

In an embodiment, a plurality of regions is defined in the torso model and step (c) comprises applying at least one constraint based on the location of the vertices in the plurality of regions. In an embodiment, the number of regions is comprised between 4 and 20. In a particular embodiment, the number of regions is 6 or 16. The division of the torso model into regions can be based on the segmentation of anatomic regions, on thresholding the distribution of vertices in the torso model or in any other approach for dividing a volume into different regions, including or not a priori anatomical information.

In an embodiment, the at least one constraint comprises one of the following options:
- an electrical axis cannot be established between vertices of the torso model belonging to the same region,
- an electrical axis must cross at least one predefined region, and/or
- an electrical axis cannot be established so as to connect a pair of vertices of the torso model belonging to predefined pairs of regions.

In an embodiment, step (d) comprises:
- computing a vector *d̅ᵢⱼ* for each pair of electrical axes *i* and *j*, being *d̅ᵢⱼ* a vector of magnitude *dᵢⱼ* and direction *d̂ᵢⱼ*, perpendicular to both electrical axes *i* and *j;* being *dᵢⱼ* the minimum distance between the electrical axes *i* and *j:* ${d}_{ij} = \left|\frac{\left({\vec{a}}_{j}-{\vec{a}}_{i}\right) ⋅ \left({\vec{b}}_{i}\times {\vec{b}}_{j}\right)}{\left|{\vec{b}}_{i}\times {\vec{b}}_{j}\right|}\right| ,$ wherein *a̅ᵢ* is a vector from the origin of coordinates to a point in electrical axis *i, d;* is a vector from the origin of coordinates to a point in electrical axis *j*, *b̅ᵢ* is a unit vector indicating the direction of electrical axis *i,* and *b̅ⱼ* is a unit vector indicating the direction of electrical axis *j*;
- computing, for each pair of electrical axes *i* and *j,* the midpoint of *d̅ᵢⱼ*; and
- defining the point of electrical symmetry as the mean point of the plurality of midpoints of *d̅ᵢⱼ* computed: ${P}_{es} = \frac{1}{I ⋅ \left(I-1\right)} {\sum }_{i} {\sum }_{j \neq i} {μ}_{ij} ,$ wherein *Pₑₛ* is the point of electrical symmetry; *µᵢⱼ =* (*xᵢⱼ, yᵢⱼ, zᵢⱼ*) is the midpoint of *d̅ᵢⱼ* for a pair of electrical axes *i* and *j; i =* 1*, ... I*; *j* = 1, ... *I*; and *I* is the total number of electrical axes.

In an embodiment, the method comprises, before step (d), defining a plurality of cylinders, each cylinder corresponding to an electrical axis and having a longitudinal axis coaxial with the electrical axis and a radius greater than 0;
wherein step (d) comprises:
- determining a total intersection volume (*V_{T}*), as the conjunction of the intersection volumes (*Vᵢⱼ*) that arise from the intersection of all the possible combinations of pairs of cylinders *i* and *j:* ${V}_{T} = {∪}_{i \neq j} {V}_{ij}$
- discretizing the total intersection volume (*V_{T}*) in voxels of a predetermined voxel size;
- quantifying the number of cylinders intersecting at each voxel of the discretized total intersection volume (*V_{T}*) and assigning the resulting value to said voxel, obtaining as a result a three-dimensional probability distribution function; and
- defining the point of electrical symmetry as the center of the voxel for which the three-dimensional probability distribution function is maximized.

In an embodiment, the method comprises processing the body surface potentials recorded at each sensor. In an embodiment, processing the body surface potentials recorded at each sensor is performed before step (c), in particular before step (b). In an embodiment, the body surface potentials are processed for eliminating at least one physiological and/or non-physiological signal component. In an embodiment, the at least one portion of the subject's heart is the upper cardiac chambers, and the body surface potentials are processed for eliminating at least one signal component associated to the lower cardiac chambers. In an embodiment, the at least one portion of the subject's heart is the lower cardiac chambers, and the body surface potentials are processed for eliminating at least one signal component associated to the upper cardiac chambers.

In a particular embodiment, the method comprises, before step (b), processing the body surface potentials recorded at each sensor for eliminating at least one physiological and/or non-physiological signal component, wherein the at least one portion of the subject's heart is the upper cardiac chambers, and the body surface potentials are processed for eliminating at least one signal component associated to the lower cardiac chambers; or the at least one portion of the subject's heart is the lower cardiac chambers, and the body surface potentials are processed for eliminating at least one signal component associated to the upper cardiac chambers.

In an embodiment, the method according to the first inventive aspect further comprises:
- providing a three-dimensional model of the at least one portion of the subject's heart; and
- locating the model of the at least one portion of the subject's heart in the determined location of the geometrical center of the at least one portion of the subject's heart.

In an embodiment, the three-dimensional model of the at least one portion of the subject's heart is obtained by one or several from the following options:
- by automatic, semi-automatic, or manual segmentation of images obtained using an imaging system, such as magnetic resonance or computerized tomography scan, wherein images are particularly images of the subject;
- from a database of previously generated cardiac models;
- generating a cardiac model from at least one mathematical model representing different subject characteristics;
- estimating a cardiac model according to any embodiment of the method according to the third inventive aspect, described hereinafter.

Particularly, in an embodiment, the three-dimensional model of the at least one portion of the subject's heart is obtained by estimating said three-dimensional model of the at least one portion of the subject's heart according to any embodiment of the method according to the third inventive aspect.

In a second inventive aspect the invention provides a computer-implemented method for determining at least one region of interest within the cardiac tissue, the method comprising the following steps:
(i) providing a three-dimensional torso model of the subject;
(ii) providing body surface potential signals measured by a plurality of sensors placed on the subject's torso, and the position of each sensor;
(iii) providing a three-dimensional model of at least one portion of the subject's heart;
(iv) locating the three-dimensional model of the at least one portion of the subject's heart at the location of the geometrical center resulting from the method according to any embodiment of the first aspect described herein;
(v) solving the inverse problem of cardiology to generate an electroanatomical map, wherein the electrical activity of each area of the at least one portion of the subject's heart is identified;
(vi) applying at least one electrocardiographic imaging (ECGI) analysis technique to the electroanatomical map; and
(vii) based on the results of the ECGI analysis, determining at least one region of interest within the cardiac tissue.

In the method according to the second inventive aspect said region of interest determined in step (vii) depends on the particular application. Non-limiting examples of regions of interest are: the region of the cardiac tissue responsible for a cardiac arrhythmia, the most adequate location for the leads of an implantable pacemaker or defibrillator, or the region of the tissue presenting anatomical or functional anomalies, such as slow conduction velocity, fibrosis or dysplasia. Particularly, the region of interest is the region of the cardiac tissue responsible for a cardiac arrhythmia.

In an embodiment, the at least one electrocardiographic imaging (ECGI) analysis technique is applied to the electroanatomical map to detect the area of the at least one portion of the subject's heart responsible for a cardiac arrhythmia. Advantageously, the present method allows to identify the regions of the at least one portion of the subject's heart responsible for a cardiac arrhythmia even at a moment when the subject is not suffering an arrhythmia.

In an embodiment of the method according to the second inventive aspect, the at least one electrocardiographic imaging analysis technique used to determine at least one region of interest within the cardiac tissue is one or several from: activation times analysis, conduction velocity analysis, phase analysis, dominant frequency analysis, driver analysis, fractionation analysis, complexity analysis, and/or voltage analysis.

In an embodiment of the method according to the second inventive aspect, the three-dimensional model of the at least one portion of the subject's heart is obtained by one or several from the following options:
- by automatic, semi-automatic, or manual segmentation of images obtained using an imaging system, such as magnetic resonance or computerized tomography scan, wherein images are particularly images of the subject;
- from a database of previously generated models;
- generating a model from at least one mathematical model representing different subject characteristics;
- estimating a cardiac model according to any embodiment of the method according to the third inventive aspect, described hereinafter.

Particularly, in an embodiment, the three-dimensional model of the at least one portion of the subject's heart is obtained by estimating said three-dimensional model of the at least one portion of the subject's heart according to any embodiment of the method according to the third inventive aspect.

In a third inventive aspect, the invention provides a computer-implemented method for estimating a three-dimensional model of at least one portion of a subject's heart, the method comprising the following steps:
- providing a basal cardiac model, *G_{H},* expressible as a function of a determined number of deformation modes, *σ̅ₘ*, as: ${G}_{H} = f \left({\overline{σ}}_{1},{\overline{σ}}_{2},…,{\overline{σ}}_{m},…,{\overline{σ}}_{M}\right) , ∀ m ∈ \left[1, M\right] ,$ being *M* the total number of deformation modes, and *σ̅ₘ* the m-th deformation mode;
- applying a weight, *αₘ*, to each deformation mode to obtain the three-dimensional model of at least one portion of the subject's heart, *G_{H}'*: ${G}_{H} ′ = f \left({α}_{1}⋅{\overline{σ}}_{1},{α}_{2}⋅{\overline{σ}}_{2},…,{α}_{m}⋅{\overline{σ}}_{m},…,{α}_{M}⋅{\overline{σ}}_{M}\right) , ∀ m ∈ \left[1, M\right] ,$ being *αₘ* the weight assigned to the m-th deformation mode.

Estimating a three-dimensional model of at least one portion of the subject's heart has an application not only in the identification of the area of the at least one portion of the subject's heart responsible for an arrhythmia, but also in other fields of cardiology unrelated to the analysis of cardiac arrhythmia, such as on the screening of cardiac activity in the general population. Since having a three-dimensional model of a subject's heart is a necessary requirement for any technology based on ECGI or in any other non-invasive means for studying cardiac activity in the surface of the heart, the methodology for obtaining a cardiac model according to the present invention is convenient for any application derived from the use of such technologies. Non-limiting examples of said applications beyond the evaluation of cardiac arrhythmia are the non-invasive evaluation of cardiac substrate without the need for medical imaging modalities, such as the identification and/or analysis of cardiac tissue fibrosis, dysplasia, slow conduction regions, etc., the non-invasive identification of the most appropriate positioning of implantable pacemakers and/or defibrillators, the evaluation of the most appropriate configuration for implantable pacemakers and/or defibrillators, or the stratification of patients in different groups attending to the non-invasive assessment of their cardiac condition.

The a priori knowledge of the heart's position within a subject's torso according to the first inventive aspect allows the possibility to avoid the use of any medical image modality on such subject on the day of the electrophysiological study. I.e., knowing the heart's position allows to incorporate a previously generated cardiac model obtained from the same subject on a different time instant by using medical imaging modalities. Beyond, the combination of the first inventive aspect with the third inventive aspect, allows to avoid the need for employing any medical imaging modality for that subject. In this way, the combination of the first and the third inventive aspects, which allows to determine the most adequate cardiac model for a given subject and the location of such within the subject's torso, results in an enabling tool for extending the use of non-invasive cardiac mapping technologies to any potential scenario in which there is no possibility, need or willingness to employ medical imaging techniques.

In an embodiment of the method according to the third inventive aspect, the basal cardiac model, *G_{H},* is constructed as an average of a population of cardiac models, the population of cardiac models comprising:
- mathematical models of hearts and/or of portions thereof, and/or
- three-dimensional models generated from the segmentation of real cardiac geometries.

In an embodiment, each mathematical model is constructed to represent determined dimensions and/or morphologies of a heart or a portion thereof.

In an embodiment, the real cardiac geometries are obtained using an imaging system.

In an embodiment of the method according to the third inventive aspect, the deformation modes of the basal cardiac model are computed by obtaining the principal components of the population of models.

In an embodiment, the principal components of the population of models are obtained using principal component analysis, independent component analysis, linear discriminant analysis, autoencoders, kernel principal component analysis and/or graph-based principal component analysis.

In an embodiment of the method according to the third inventive aspect, the weights, *αₘ*, applied to each deformation mode to obtain the three-dimensional model of at least one portion of the subject's heart are obtained from information representative of the cardiac structure of the subject, at least one demographic feature of the subject and/or at least one pathological feature of the subject. In an embodiment, the information representative of the cardiac structure of the subject comprises the dimensions of at least one portion of the heart and/or at least one functional parameter, such as the degree of hypertrophy or the ejection fraction.

In an embodiment of the method according to the third inventive aspect:
- a plurality of estimated cardiac models, ${G}_{H}^{i}$, is obtained from the basal cardiac model, *G_{H},* by applying a plurality of combinations of weights of the deformation modes to the basal cardiac model, *G_{H}*;
- for each estimated cardiac model, ${G}_{H}^{i}$, a transfer matrix, ***Mⁱ***, is estimated based on the relationship between the three-dimensional torso model and the estimated cardiac model, ${G}_{H}^{i}$;
- for each estimated cardiac model, ${G}_{H}^{i}$, the inverse problem: ${M}^{i} {U}_{H}^{i} = {U}_{T}$ is solved,
   ***Mⁱ*** being the estimated transfer matrix, ${U}_{H}^{i}$ being the electrical activity at the cardiac surface of estimated cardiac model, ${G}_{H}^{i}$, and ***U**_{T}* being the body surface potentials at the torso surface;
- the three-dimensional model of at least one portion of the subject's heart, *G_{H}',* is selected from the plurality of estimated cardiac models, ${G}_{H}^{i}$, as the estimated cardiac model which satisfies a predefined condition, such as the minimization of a loss function or the maximization of a similarity metric (e.g., the maximization of the similarity between the acquired body surface potentials, and the body surface potentials obtained when propagating a mathematical simulation of cardiac signals from the surface of the estimated cardiac model to the surface of the torso).

In an embodiment of the method according to the third inventive aspect, for each estimated cardiac model, ${G}_{H}^{i}$, the inverse problem is solved by minimizing the following equation: ${\left‖{M}^{i}{U}_{H}^{i}-{U}_{T}\right‖}^{2} + {λ}^{i} {\left‖{B}^{i}{U}_{H}^{i}\right‖}^{2} ,$ where *λⁱ* is a regularization parameter and ***Bⁱ*** is a spatial regularization matrix.

In an embodiment of the method according to the third inventive aspect, Tikhonov regularization and the L-curve method are used to select *λⁱ* for each estimated cardiac model, ${G}_{H}^{i}$, *λⁱ* being selected as the value corresponding to the maximal curvature of the L-curve; and the three-dimensional model of at least one portion of the subject's heart, *G_{H}',* being selected as the estimated cardiac model for which the maximum curvature of the L-curve is obtained.

In an embodiment of the method according to the third inventive aspect, for each estimated cardiac model, ${G}_{H}^{i}$, the inverse problem is solved by minimizing the following equation: ${\left‖{M}^{i}{U}_{H}^{i}-{U}_{T}\right‖}^{2} + {λ}^{i} {\left‖{B}^{i}{U}_{H}^{i}\right‖}^{2} ,$ **where** *λⁱ* **is** a regularization parameter and ***Bⁱ*** is a spatial regularization matrix, wherein the Tikhonov regularization and the L-curve method are used to select *λⁱ* for each estimated cardiac model, ${G}_{H}^{i}$, *λⁱ* being selected as the value corresponding to the maximal curvature of the L-curve; and the three-dimensional model of at least one portion of the subject's heart, *G_{H}',* being selected as the estimated cardiac model for which the maximum curvature of the L-curve is obtained.

In a fourth inventive aspect, the invention provides a method for determining the location within the torso and the morphology of at least one portion of a subject's heart, the method comprising:
- determining the location of the at least one portion of the subject's heart within the torso according to the method of the first inventive aspect,
- determining the three-dimensional model of the at least one portion of the subject's heart according to the method of the third inventive aspect, and
- locating the three-dimensional model of the at least one portion of the subject's heart in the location of the geometrical center determined according to the method of the first inventive aspect.

In an embodiment, the method according to the fourth inventive aspect further comprises the following steps:
- solving the inverse problem of cardiology to generate an electroanatomical map, wherein the electrical activity of each area of the at least one portion of the subject's heart is identified;
- applying at least one electrocardiographic imaging (ECGI) analysis technique to the electroanatomical map; and
- based on the results of the ECGI analysis, determining at least one region of interest within the cardiac tissue.

In an embodiment, said region of interest depends on the particular application. Non-limiting examples of regions of interest are: the region of the cardiac tissue responsible for a cardiac arrhythmia, the most adequate location for the leads of an implantable pacemaker or defibrillator, or the region of the tissue presenting anatomical or functional anomalies, such as slow conduction velocity, fibrosis or dysplasia.

### DESCRIPTION OF THE DRAWINGS

These and other characteristics and advantages of the invention will become clearly understood in view of the detailed description of the invention which becomes apparent from particular embodiments of the invention, given just as examples and not being limited thereto, with reference to the drawings.
- FIG. 1: This figure shows a block diagram of the steps followed for obtaining the appropriate location of at least one portion of a subject's heart inside a torso in a particular embodiment of the present invention.
- FIG. 2: This figure shows a block diagram of the acquisition of input data of a particular embodiment of the present invention.
- FIG. 3A-3B: These figures show, respectively, an example of a three-dimensional model of a portion of a heart, particularly of the upper chambers of the heart (or atria), and a three-dimensional model of a torso.
- FIG. 4: This figure shows an example of the basic elements of a three-dimensional mesh or geometric model.
- FIG. 5A-5B: These figures show, respectively, the definition of an electrical axis between two vertices, A and A', of a torso model, and the body surface potentials obtained at such vertices.
- FIG. 6A-6B: These figures show, respectively, the definition of regions in the torso model according to two possible embodiments.
- FIG. 7: This figure shows the definition of a vector *r̅ᵢ* which describes any point of a straight line.
- FIG. 8: This figure shows the minimum distance between two straight lines, and the midpoint of a vector which is perpendicular to both straight lines and whose magnitude is the minimum distance between them.
- FIG. 9A-9B: These figures show, respectively, the definition of a cylinder with radius greater than 0, and the intersection volume between two cylinders.
- FIG. 10: This figure shows the discrete three-dimensional probability distribution function generated from the intersection between all the possible cylinders in an embodiment of the invention.
- FIG. 11: This figure shows the location of a cardiac geometrical model within a torso model. The cardiac model is located at the location indicated by the geometrical center determined according to an embodiment of the invention.
- FIG. 12A-12B: These figures show examples of a modification of the weights of the deformation modes of a three-dimensional model to vary its shape. FIG. 12A represents a model of a sphere, with three deformation modes, *σ̅*₁*, σ̅*₂ and σ̅₃. FIG. 12B represents the shape variation in the model of FIG 12A by applying different weights to its deformation modes.
- FIG. 13: This figure shows a block diagram of the steps followed for obtaining the appropriate location and morphology of the heart inside a torso in a particular embodiment of the present invention.

### DETAILED DESCRIPTION OF THE INVENTION

According to a first inventive aspect, the present invention defines a computer-implemented method for determining the location of at least one portion of a subject's heart within the subject's torso, the method comprising the following steps:
(a) providing:
   - a three-dimensional torso model (4) of the subject, the torso model (4) being defined by a plurality of vertices (12) and a plurality of faces (13), each of said faces (13) determined by at least three vertices (12) and at least three edges (14), each edge (14) connecting a pair of vertices (12),
   - the position (5) of each sensor of a plurality of sensors placed on the subject's torso, and
   - a body surface potential (8) measured by each sensor;
(b) interpolating (16) the body surface potential (8) in each vertex (12) of the torso model (4) based on the body surface potentials (8) measured by the sensors;
(c) defining a plurality of electrical axes (17), each electrical axis (17) being an imaginary straight line connecting a vertex (12) with another vertex (12), the body surface potential (8) of which has the greatest morphological similarity but inverse polarity;
(d) determining the location of a point of electrical symmetry (3) in the torso model (4) based on the plurality of electrical axes (17); and
(e) determining the location of the geometrical center (2) of the at least one portion of the subject's heart in the torso model (4) based on the determined point of electrical symmetry (3).

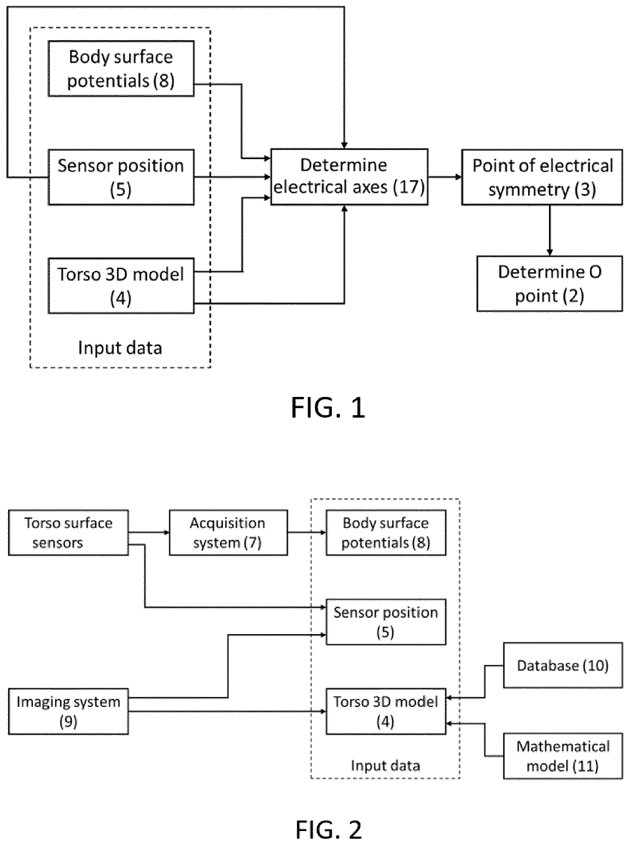

FIG. 1 shows a block diagram of the steps followed for obtaining the appropriate location of at least one portion of a subject's heart inside a torso in a particular embodiment of the present invention.

### Obtention of input data

The method of the present invention for determining the location of at least one portion of a subject's heart within a subject's torso requires input data, said input data comprising a three-dimensional model (4) of the subject's torso, a plurality of body surface potentials (8) acquired in the surface of the subject's torso and the position (5) of the sensors used to acquire said body surface potentials (8) on the surface of the subject's torso.

FIG. 2 shows a block diagram of the acquisition of input data of a particular embodiment of the present invention.

In an embodiment of the invention, the required input data is obtained by following a series of steps, said steps comprising:
- Disposing a plurality of sensors on the subject's torso surface;
- Obtaining a three-dimensional model (4) of the subject's torso;
- Determining the position (5) of each sensor on the subject's torso; and
- Acquiring the body surface potential (8) at each sensor on the subject's torso.

In some embodiments of the invention, the plurality of sensors placed on the subject's torso surface comprise from 100 to 150 electrodes. In an embodiment, the sensors are homogeneously distributed to cover the whole torso surface. In a particular embodiment, the plurality of sensors placed on the subject's torso comprises 128 electrodes homogeneously distributed to cover the whole torso surface.

In an embodiment of the invention, the employed sensors are conventional electrodes for the acquisition of body surface potentials, e.g., electrodes for the acquisition of surface electrocardiographic signals. In other embodiments, the plurality of sensors is arranged on a sensor vest specifically designed for the acquisition of body surface potentials.

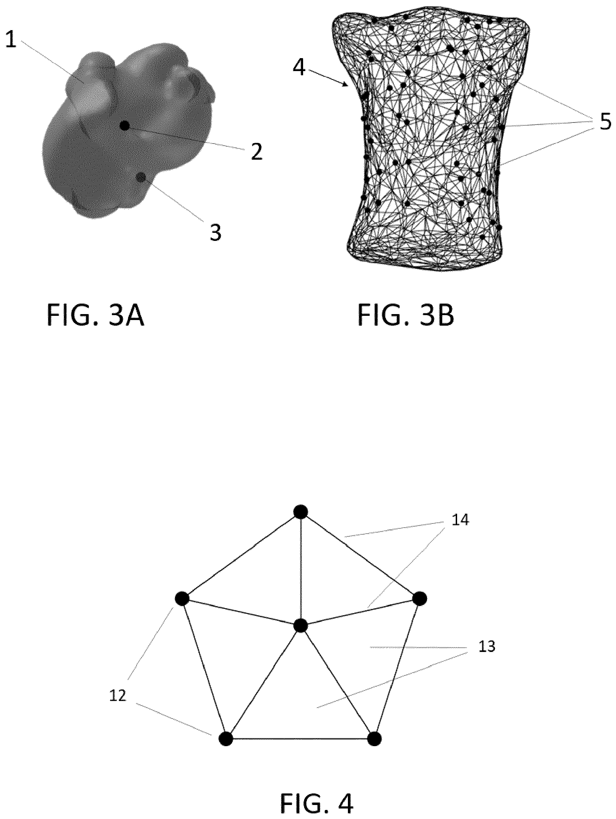

FIGs. 3A-3B show, respectively, an example of a three-dimensional model of a portion of a heart (1), particularly of the upper chambers of the heart (or atria), showing the geometrical center (2) and the point of electrical symmetry (3) of the portion of a heart; and a three-dimensional model (4) of a torso showing the position (5) of each sensor.

In the context of the invention, a three-dimensional model (4) of the torso is a convex hull comprising a plurality of vertices (12) and a plurality of faces (13), each of said faces (13) determined by at least three vertices (12) and at least three edges (14) connecting each pair of them. Particularly, each of said faces is determined by three vertices (13) and three edges (14) connecting each pair of vertices (12). FIG. 4 shows an example of the basic elements of a three-dimensional mesh or geometric model.

In embodiments of the invention, the three-dimensional model (4) of the subject's torso is obtained by automatic, semi-automatic, or manual segmentation of images obtained using an imaging system (9). Particularly, images are images of the subject. In a particular embodiment, the imaging system is a medical imaging system such as magnetic resonance or computerized tomography scan. In another particular embodiment, the images are non-medical images of the torso obtained using non-limiting examples of non-medical imaging modalities such as photogrammetry, video recordings or speckle interferometry.

In a particular embodiment, the torso model is obtained by automatic, semi-automatic, or manual segmentation of images, wherein the images are non-medical images, such as photogrammetry, video recordings or speckle interferometry. Particularly, images are at least two 2D images obtained using a conventional camera.

In an embodiment, the torso model is obtained by automatic segmentation of images obtained using a conventional camera.

In yet another particular embodiment, the three-dimensional model of the subject's torso (4) is obtained from a database (10) of previously generated torso models (4).

In yet another particular embodiment, the three-dimensional model (4) of the subject's torso is generated from one or several mathematical models (11) representing different subject characteristics. Particularly, the different subject characteristics can be selected from the group consisting of height, weight, age, sex, race, disease statuses or any other measurable parameter.

In an embodiment, the torso model of the subject is determined as part of the method according to the first inventive aspect. In another embodiment, the torso model has been previously determined and stored in non-limiting examples of computer readable mediums such as electronic media, e.g. flash memories, optical media, e.g. CD-ROMs, magnetic media, e.g. hard disks or floppy disks, or any other storage media which is intended to be read by a computer. In this way, in step a) of the method the torso model can be loaded from any of the disclosed storage modalities.

The method according to the first inventive aspect requires to know the precise location (5) of each sensor on the torso surface to determine the location at which the body surface potential (8) is measured.

In embodiments of the invention, the sensor location (5) is obtained by automatic, semi-automatic, or manual segmentation of images obtained using an imaging system (9). Particularly, images are images of the subject. In one embodiment, the sensor location (5) is obtained by automatic, semi-automatic, or manual segmentation of medical images of the torso obtained using medical imaging modalities. Particularly, the medical imaging modalities can be magnetic resonance or computerized tomography scan. In such embodiments, sensors containing radio-opaque materials or contrast agents can be used to facilitate their location using medical imaging systems. In another embodiment, the sensor location (5) is obtained by automatic, semi-automatic, or manual segmentation of non-medical images of the torso obtained using non-medical imaging modalities. Particularly, the non-medical imaging modalities can be photogrammetry or video recordings. In a particular embodiment, the sensor location (5) is obtained by automatic, semi-automatic, or manual segmentation of non-medical images of the torso obtained using a conventional camera.

In an embodiment, the position of each sensor on the subject's torso is determined by automatic segmentation of images using an imaging system.

In an embodiment, the position of each sensor on the subject's torso is determined by automatic segmentation of images, wherein the images are non-medical images, such as photogrammetry or video recordings. Particularly, images are at least two 2D images obtained using a conventional camera.

In a particular embodiment, the employed sensors can contain human-readable or non-human-readable codes, labels and/or drawings to facilitate the use of artificial intelligence-based approaches for the identification of their location (5).

In an embodiment, the positions of the sensors are determined as part of the method according to the first inventive aspect. In another embodiment, the positions of the sensors have been previously determined and stored in non-limiting examples of computer readable mediums such as electronic media, e.g. flash memories, optical media, e.g. CD-ROMs, magnetic media, e.g. hard disks or floppy disks, or any other storage media which is intended to be read by a computer. In this way, in step a) of the method the positions of the sensors can be loaded from any of the disclosed storage modalities.

The method according to the first inventive aspect also requires the provision of body surface potentials (8) acquired at a plurality of locations (5) on the subject's torso surface, i.e. the provision of the electrical activity measured at each sensor on the subject's torso.

In some embodiments, the sensors are connected to an acquisition system (7) through electrically conductive pathways. In particular embodiments, the acquisition system (7) can be a bipotential amplifier. The acquisition system (7) can amplify and digitize the acquired body surface potentials (8), so that they can be analyzed by a digital system. Particularly, the digital system can be a computer device such as PC, laptop, tablet or smartphone, or a processor, FPGA, integrated circuit, or server, among others. The term "computer device" used herein refers to any computer hardware with a central processing unit (CPU) which can also include a memory and/or a database.

In some embodiments of the invention, the electrical pathways connecting the sensors to the acquisition system (7) can be e.g., cables, conductive ink pathways or any other type of electrical conductor allowing to transfer the body surface potentials (8) measured by the sensors to the acquisition system (7).

FIG. 3B shows a three-dimensional model (4) of a torso, wherein the position (5) of the sensors is also schematically depicted.

In an embodiment, the body surface potentials are measured as part of the method according to the first inventive aspect. In another embodiment, the body surface potentials have been previously determined and stored in non-limiting examples of computer readable mediums such as electronic media, e.g. flash memories, optical media, e.g. CD-ROMs, magnetic media, e.g. hard disks or floppy disks, or any other storage media which is intended to be read by a computer. In this way, in step a) of the method the body surface potentials can be loaded from any of the disclosed storage modalities.

### Determining heart location

The method for determining the location of the at least one portion of the subject's heart within a subject's torso comprises:
(b) interpolating (16) the body surface potential (8) in each vertex (12) of the torso model (4) based on the body surface potentials (8) measured by the sensors;
(c) defining a plurality of electrical axes (17), each electrical axis (17) being an imaginary straight line connecting a vertex (12) with another vertex (12), the body surface potential (8) of which has the greatest morphological similarity but inverse polarity;
(d) determining the location of a point of electrical symmetry (3) in the torso model (4) based on the plurality of electrical axes (17); and
(e) determining the location of the geometrical center (2) of the at least one portion of the subject's heart in the torso model (4) based on the determined point of electrical symmetry (3).

In one embodiment, the location of the sensors (5) matches with some of the vertices (12) of the torso model (4). The number of sensors can be lower than the total number of vertices (12) in the torso model (4). In a particular embodiment, the number of vertices (12) in the torso geometry (4) corresponding to sensors is one order of magnitude less than the total number of vertices (12) in the torso model (4). In another particular embodiment, the number of vertices (12) in the torso model (4) corresponding to sensors is two orders of magnitude less than the total number of vertices (12) in the torso model (4).

In an embodiment of the invention, the body surface potential (8) in each vertex (12) of the torso geometry (4) is obtained by measuring at the location of said vertex (12) and/or by interpolating (16) at said vertex (12) the body surface potentials (8) measured at other positions in order to increase the spatial resolution of the surface potential distribution on the torso.

In an embodiment of the invention, the interpolation (16) technique is Laplacian interpolation, Radial Basis Function interpolation, Cubic Splines interpolation, Nonmanifold Laplacian interpolation, or any other interpolation approach suitable for geometric meshes. In a particular embodiment, Nonmanifold Laplacian interpolation is used for interpolation (16).

Once the body surface potential (8) at each vertex (12) has been measured and/or interpolated (16), a plurality of electrical axes (17) is defined. In the context of the present invention, electrical axes (17) are defined as the imaginary straight lines connecting the two vertices of the torso model (4) whose body surface potentials (8) have the greatest morphological similarity, but with inverse polarity. In one embodiment, each vertex (12) of the torso geometry is paired with the vertex (12) whose body surface potential (8) has the greatest negative correlation with respect to its own body surface potential (8), which is the indicator of inverse polarity within each pair of vertices (12). In one embodiment of the invention, the correlation between vertices is calculated for all pairs of vertices (12) on the torso model (4).

In other embodiment, each vertex (12) of the torso geometry is paired with the vertex (12) whose body surface potential (8) has the lowest least squares value with respect to its own body surface potential (8), both paired vertices having inverse polarity. In one embodiment of the invention, the least squares value between vertices is calculated for all pairs of vertices (12) on the torso model (4).

In other embodiment, each vertex (12) of the torso geometry is paired with the vertex (12) whose body surface potential (8) has the lowest dynamic time warping deformation with respect to its own body surface potential (8), both paired vertices having inverse polarity. In one embodiment of the invention, the dynamic time warping deformation between vertices is calculated for all pairs of vertices (12) on the torso model (4).

In other embodiment, each vertex (12) of the torso geometry is paired with the vertex (12) whose body surface potential (8) has the greatest negative covariance with respect to its own body surface potential (8), which is the indicator of inverse polarity within each pair of vertices (12). In one embodiment of the invention, the covariance between vertices is calculated for all pairs of vertices (12) on the torso model (4).

In other embodiment, each vertex (12) of the torso geometry is paired with the vertex (12) whose body surface potential (8) has the lowest vector distance with respect to its own body surface potential (8), both paired vertices having inverse polarity. In one embodiment of the invention, the vector distance between vertices is calculated for all pairs of vertices (12) on the torso model (4).

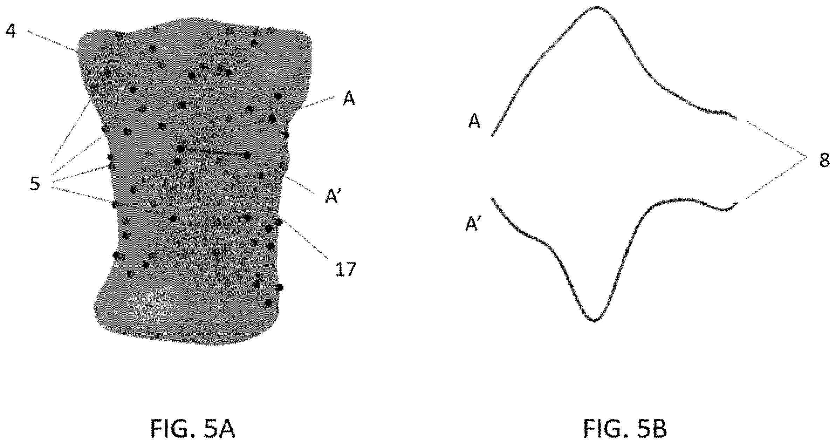

An example of the calculation of electrical axes is depicted in FIGs. 5A-5B. In FIG. 5A an electrical axis (17) has been defined between two vertices identified as A and A' in the figure. The body surface potential (8) at vertices A and A' is shown in FIG. 5B. In particular, the potentials displayed in FIG. 5B correspond to the P waves of surface ECG signals. As visible from FIG. 5B, the body surface potentials at vertices A and A' have great morphological similarity but inverse polarity.

In an embodiment, an electrical axis is defined between two vertices only if the correlation between their body surface potentials reaches a predefined threshold. According to this embodiment, the vertices having a body surface potential which does not comply with a minimum correlation threshold with the body surface potential of any other vertex are kept disconnected, such that no electrical axis is defined between them.

A vertex can be part of more than one electrical axis if its body surface potential has the greatest negative correlation with the body surface potential of more than one vertex.

In an embodiment, an electrical axis is defined between two vertices only if the least squares value between their body surface potentials is below a predefined threshold. According to this embodiment, the vertices having a body surface potential which does not comply with a maximum least squares threshold with the body surface potential of any other vertex are kept disconnected, such that no electrical axis is defined for them.

A vertex can be part of more than one electrical axis if its body surface potential has the lowest least squares value with the body surface potential of more than one vertex, the body surface potentials having inverse polarity.

In an embodiment, an electrical axis is defined between two vertices only if the dynamic time warping deformation between their body surface potentials is below a predefined threshold. According to this embodiment, the vertices having a body surface potential which does not comply with a maximum deformation threshold with the body surface potential of any other vertex are kept disconnected, such that no electrical axis is defined for them.

A vertex can be part of more than one electrical axis if its body surface potential has the lowest dynamic time warping deformation with the body surface potential of more than one vertex, the body surface potentials having inverse polarity.

In an embodiment, an electrical axis is defined between two vertices only if the covariance between their body surface potentials reaches a predefined threshold. According to this embodiment, the vertices having a body surface potential which does not comply with a minimum covariance threshold with the body surface potential of any other vertex are kept disconnected, such that no electrical axis is defined for them.

A vertex can be part of more than one electrical axis if its body surface potential has the greatest negative covariance with the body surface potential of more than one vertex.

In an embodiment, an electrical axis is defined between two vertices only if the vector distance between their body surface potentials is below a predefined threshold. According to this embodiment, the vertices having a body surface potential which does not comply with a maximum distance threshold with the body surface potential of any other vertex are kept disconnected, such that no electrical axis is defined for them.

A vertex can be part of more than one electrical axis if its body surface potential has the lowest vector distance with the body surface potential of more than one vertex, the body surface potentials having inverse polarity.

Based on the plurality of electrical axes (17) the location of a point of electrical symmetry (3) in the torso model (4) is determined. The location of the geometrical center (2) of the at least one portion of the subject's heart in the torso model (4) is determined based on the determined point of electrical symmetry (3).

In an embodiment of the invention, the electrical signals corresponding to the body surface potentials (8) acquired at each sensor on the subject's torso undergo one or more signal processing (15) stages, for example for eliminating non-physiological signal components such as baseline wander, muscle noise, and/or powerline interferences. Additionally or alternatively, certain physiological components can be removed in order to improve the precision in the location of the O point or geometrical center (2). One non-limiting example is the separated analysis of either atrial or ventricular activity, in which the physiological components of the electrical signal that are not to be analyzed are removed in advance. For example, in the analysis of atrial activity, the QRST component of the surface electrical signals (8) can be removed by using QRST cancellation techniques.

In an embodiment of the invention, the signal processing (15) stages for eliminating physiological and/or non-physiological signal components are performed before step (c), in particular before step (b).

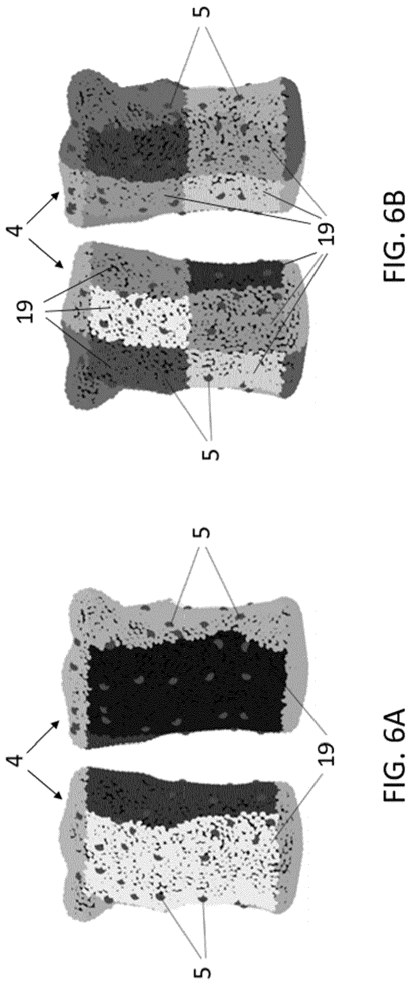

In an embodiment of the invention, a plurality of regions (19) is defined in the torso model (4) and step (c) comprises applying at least one constraint based on the location of the vertices (12) in the plurality of regions (19). FIGs. 6A-6B show, respectively, the definition of regions (19) in the torso model (4) according to two possible embodiments, wherein the torso model (4) is divided into 4 and 16 regions (19), respectively. In these figures, the different regions (19) are schematically depicted in different tones of grey. In the embodiments shown the division of the torso model (4) into regions (19) has been performed based on the segmentation of anatomic regions. However, other approaches can be used to define the plurality of regions (19) in the torso model (4). Also, a different number of regions (19) can be defined in other embodiments.

In an embodiment of the invention, the constraint applied to the electrical axes (17) comprises one or several of the following options:
- an electrical axis (17) cannot be established between vertices (12) of the torso model (4) belonging to the same region (19);
- an electrical axis (17) must cross at least one predefined region (19);
- an electrical axis (17) cannot be established so as to connect a pair of vertices (12) of the torso model (4) belonging to predefined pairs of regions (19), such as pairs of regions (19) that would imply that the electrical axis (17) does not cross through anatomically possible cardiac locations.

In the example of FIG. 6A a possible constraint can be established according to which electrical axes (17) are not allowed between vertices (12) located in same region (19). In the example of FIG. 6B the same constraint can be applied. Additionally or alternatively, in the example of FIG. 6B a possible constraint can be established according to which electrical axes (17) are not allowed to connect vertices (12) located in the abdominal region with vertices (12) located in the lumbar region.

In an embodiment of the invention, the point of electrical symmetry (3) of the at least one portion of the subject's heart, i.e. the electrical center of said at least one portion of the subject's heart, is determined as the intersection between all the electrical axes (17). In some embodiments, only a portion of the heart, such as the upper or lower chambers of the heart, is of interest, and the point of electrical symmetry (3) refers only to said portion. When the whole heart is of interest, the point of electrical symmetry (3) refers to the whole heart.

In a particular embodiment of the invention, the electrical axes (17) intersect and the point of electrical symmetry (3) is determined as the intersection of the electrical axes (17).

In a particular embodiment of the invention, the electrical axes (17) do not necessarily intersect, given that the torso model (4) and the acquisition (7) of the body surface potentials (8) are discretized to a finite number of vertices (12), and to the different electrical pathways which can be followed by cardiac potentials due to heterogeneity in tissue and conductivity along the torso. In such embodiment, the point of electrical symmetry (3) can be set within a region determined by all the electrical axes (17). In an embodiment of the invention, to estimate the precise location of the point of electrical symmetry (3), a point equidistant to a pair of electrical axes is calculated for each pair of electrical axes (17), at a distance being the minimum possible between each pair of electrical axes (17).

Generally, any point of a straight line can be described by a vector, *̅r̅*̅, which can be defined in the three-dimensional space as: $\vec{r} = \vec{a} + λ \vec{b} ,$ being *̅a̅*̅ a vector from the origin of coordinates to a point *r* in the straight line, *̅b̅*̅ a unit vector indicating the direction of the straight line, and *λ* a constant.

In an embodiment of the invention, two different points in two different electrical axes i and *j* are identified with two vectors, *r̅ᵢ* and *r̅ⱼ*, defined respectively as: ${\vec{r}}_{i} = {\vec{a}}_{i} + {λ}_{i} {\vec{b}}_{i ,}$ ${\vec{r}}_{j} = {\vec{a}}_{j} + {λ}_{j} {\vec{b}}_{j} .$

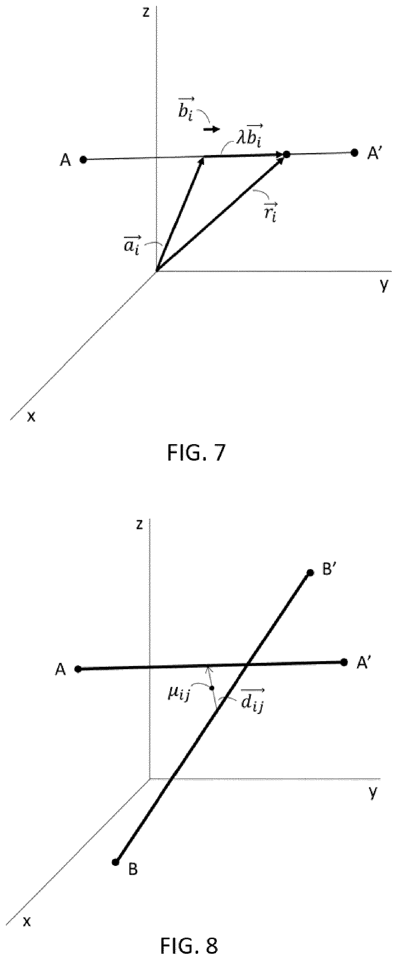

FIG. 7 shows the definition of vector *r̅ᵢ* which describes any point of electrical axis *i*, wherein electrical axis *i* connects vertices A and A'.

The minimum distance *dᵢⱼ* between the electrical axes defined by *r̅ᵢ* and *r̅ⱼ* can be defined as: ${d}_{ij} = \left|\frac{\left({\vec{a}}_{j}-{\vec{a}}_{i}\right) ⋅ \left({\vec{b}}_{i}\times {\vec{b}}_{j}\right)}{\left|{\vec{b}}_{i}\times {\vec{b}}_{j}\right|}\right| ,$ wherein *a̅ᵢ* is a vector from the origin of coordinates to a point in electrical axis *i, a̅ⱼ* is a vector from the origin of coordinates to a point in electrical axis *j, b̅ᵢ* is a unit vector indicating the direction of electrical axis *i,* and *b̅ⱼ* is a unit vector indicating the direction of electrical axis *j*, being *d̅ᵢⱼ = dᵢⱼ · d̂ᵢⱼ*, a vector of magnitude *dᵢⱼ* and direction *d̂ᵢⱼ* which is perpendicular to both electrical axes (17).

In a particular embodiment of the invention, a vector *d̅ᵢⱼ* is computed for each pair of electrical axes *i* and *j,* being *d̅ᵢⱼ* perpendicular to both electrical axes *i* and *j.* The midpoint of *d̅ᵢⱼ, µᵢⱼ* = (*xᵢⱼ, yᵢⱼ, zᵢⱼ*), is computed for each pair of electrical axes *i* and *j,* and the point of electrical symmetry (3) is determined as: ${P}_{es} = \frac{1}{I ⋅ \left(I-1\right)} {\sum }_{i} {\sum }_{j \neq i} {μ}_{ij} ,$ **wherein** *Pₑₛ* is the point of electrical symmetry; *µᵢⱼ =* (*xᵢⱼ*, *yᵢⱼ, zᵢⱼ*) is the midpoint of *d̅ᵢⱼ* for a pair of electrical axes *i* and *j*; *i =* 1*, ... I*; *j* = 1, ... *I*; and *I* is the total number of electrical axes (17).

FIG. 8 shows electrical axis *i* defined between vertices A and A' and electrical axis *j* defined between vertices B and B'. FIG. 8 also shows the minimum distance *dᵢⱼ* between electrical axes *i* and *j,* and the midpoint *µᵢⱼ* of a vector *d̅ᵢⱼ* which is perpendicular to both electrical axes and whose magnitude is the minimum distance between them.

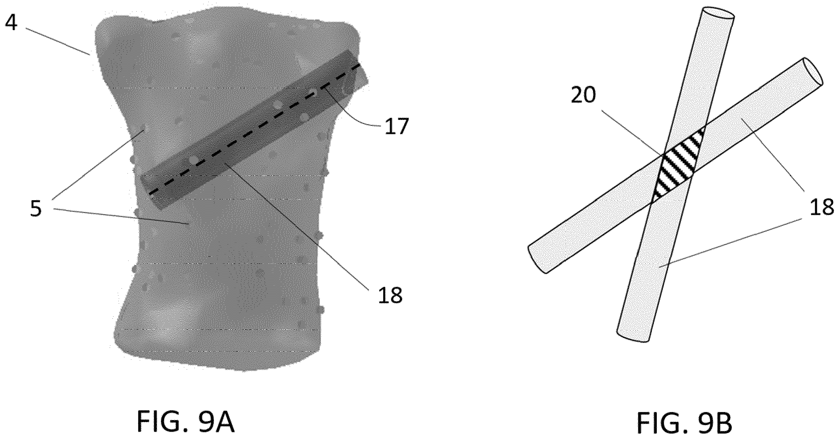

In another embodiment, the method according to the first inventive aspect comprises, before step (d), defining a plurality of cylinders (18) based on the plurality of electrical axis (17). In particular, for each electrical axis (17) a cylinder is defined having a longitudinal axis coaxial with the electrical axis (17) and a radius greater than 0. FIG. 9A shows the definition of a cylinder with radius greater than 0, as well as the corresponding electrical axis (17) which defines the longitudinal axis of the cylinder (18). In an embodiment, the radius has a value less than or equal to 100 mm. In a particular embodiment, the radius of the cylinders is 50 mm.

In the embodiment wherein a plurality of cylinders (18) is defined, cylinders (18) do not intersect each other in a point, but there is an intersection volume (20) instead. FIG. 9B shows the intersection volume (20) between two cylinders (18). The total intersection volume, *V_{T},* can be defined as the conjunction of the intersection volumes *Vᵢⱼ* that arise of the intersection of all the possible combinations of two cylinders *i* and *j.*

In an embodiment, step (d) comprises:
- determining a total intersection volume (*V_{T}*) as the conjunction of the intersection volumes (*Vᵢⱼ*) that arise from the intersection of all the possible combinations of pairs of cylinders *i* and *j:* ${V}_{T} = {∪}_{i \neq j} {V}_{ij}$
- discretizing the total intersection volume (*V_{T}*) in voxels of a predetermined voxel size;
- quantifying the number of cylinders intersecting at each voxel of the discretized total intersection volume (*V_{T}*) and assigning the resulting value to said voxel, obtaining as a result a three-dimensional probability distribution function (21); and
- defining the point of electrical symmetry (3) as the center of the voxel for which the three-dimensional probability distribution function (21) is maximized.

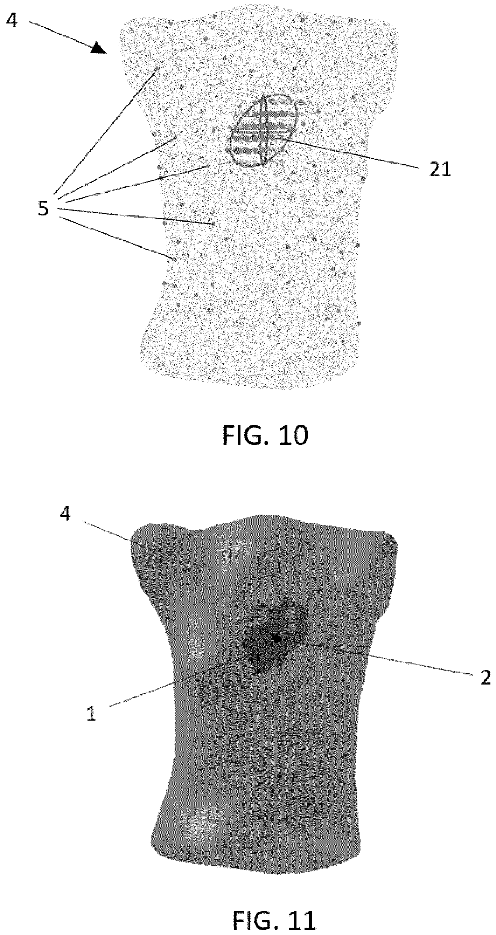

FIG. 10 shows the discrete three-dimensional probability distribution function (21) generated from the intersection between all the possible cylinders. Such probability distribution represents the odds of each voxel to be the point of electrical symmetry (3) of the at least one portion of the subject's heart. In this particular embodiment, the point of electrical symmetry (3) is defined as the center of the voxel for which the described probability distribution function (21) is maximized.

In an embodiment of the invention, the geometrical center (2) of the at least one portion of the subject's heart is set as the determined point of electrical symmetry (3).

In another embodiment of the invention, the geometrical center (2) of the at least one portion of the subject's heart is computed based on the determined point of electrical symmetry (3), namely by applying an offset correction to the position of the point of electrical symmetry (3). In a particular embodiment, the offset correction is estimated from particular characteristics of the subject, such as physical or electrical characteristics. In another particular embodiment, the offset correction is estimated from a population of subjects with known geometrical (2) and electrical centers (3) of the at least one portion of the heart.

In some embodiments, only a portion of the heart (such as the upper or lower chambers of the heart) is of interest and the geometrical center (2) and the point of electrical symmetry (3) refer only to said particular portion of interest. In such embodiments of the invention, the geometrical center (2) of the portion of the heart can be computed by applying an offset correction to the position of electrical symmetry (3), such offset correction being not necessarily the same needed to calculate the geometrical center (2) of the whole heart.

In an embodiment, the method according to the first inventive aspect further comprises:
- providing a three-dimensional model of the at least one portion of the subject's heart (1); and
- locating the model of the at least one portion of the subject's heart (1) in the determined location of the geometrical center (2).

FIG. 11 shows the location of a three-dimensional model of the at least one portion of the subject's heart (1) within a torso model, more specifically at the position indicated by the determined geometrical center (2).

The provision of a three-dimensional model of the at least one portion of the subject's heart (1) can be performed in different ways. In an embodiment, the model of the at least one portion of the subject's heart (1) is estimated using any of the embodiments of the method according to the third inventive aspect of the invention. In other embodiments, the three-dimensional model of the at least one portion of the subject's heart (1) is obtained by automatic, semi-automatic, or manual segmentation of images obtained using an imaging system (9). Particularly, images are images of the subject. In one embodiment, the three-dimensional model of the at least one portion of the subject's heart (1) is obtained by automatic, semi-automatic, or manual segmentation of medical images of the torso obtained using non-limiting examples of medical imaging modalities, such as magnetic resonance or computerized tomography scan. In other embodiments, the three-dimensional model of the at least one portion of the subject's heart (1) is obtained from a database (10) of previously generated models or generated from mathematical models (11) representing different subject characteristics. Particularly, the different subject characteristics can be selected from the group consisting of height, weight, age, sex, race, disease statuses or any other measurable parameter.

In an embodiment, the model of the at least one portion of the subject's heart is estimated as part of the method according to the first inventive aspect. In another embodiment, the model of the at least one portion of the subject's heart has been previously determined and stored in non-limiting examples of computer readable mediums such as electronic media, e.g. flash memories, optical media, e.g. CD-ROMs, magnetic media, e.g. hard disks or floppy disks, or any other storage media which is intended to be read by a computer. In this way, the model of the at least one portion of the subject's heart can be loaded from any of the disclosed storage modalities.

### Determination of at least one region of interest within the cardiac tissue

According to a second inventive aspect of the invention, a computer-implemented method for determining at least one region of interest within the cardiac tissue is provided, the method comprising the following steps:
(i) providing a three-dimensional torso model (4) of the subject;
(ii) providing body surface potentials (8) measured by a plurality of sensors placed on the subject's torso, and the position (5) of each sensor;
(iii) providing a three-dimensional model of at least one portion of the subject's heart (1);
(iv) locating the three-dimensional model of the at least one portion of the subject's heart (1) at the location of the geometrical center (2) resulting from the method according to the first inventive aspect;
(v) solving the inverse problem of cardiology to generate an electroanatomical map, wherein the electrical activity of each area of the at least one portion of the subject's heart is identified;
(vi) applying at least one electrocardiographic imaging (ECGI) analysis technique to the electroanatomical map; and
(vii) based on the results of the ECGI analysis, determining at least one region of interest within the cardiac tissue.

In the method according to the second inventive aspect, said region of interest determined in step (vii) depends on the particular application. Non-limiting examples of regions of interest are: the region of the cardiac tissue responsible for a cardiac arrhythmia, the most adequate location for the leads of an implantable pacemaker or defibrillator, or the region of the tissue presenting anatomical or functional anomalies, such as slow conduction velocity, fibrosis or dysplasia.

The provision of a three-dimensional model of the at least one portion of the subject's heart (1) can be performed in different ways. In an embodiment, the model of the at least one portion of the subject's heart (1) is estimated using any of the embodiments of the method according to the third inventive aspect of the invention. In other embodiments, the three-dimensional model of the at least one portion of the subject's heart (1) is obtained by automatic, semi-automatic, or manual segmentation of images obtained using an imaging system (9), for example using non-limiting examples of medical imaging modalities, such as magnetic resonance or computerized tomography scan. Particularly, images are images of the subject. In other embodiments, the three-dimensional model of the at least one portion of the subject's heart (1) is obtained from a database (10) of previously generated models or generated from mathematical models (11) representing different subject characteristics. Particularly, the different subject characteristics can be selected from the group consisting of height, weight, age, gender, race, disease statuses or any other measurable parameter.

In an embodiment, the three-dimensional model of the at least one portion of the subject's heart and/or the three-dimensional torso model is determined as part of the method according to the second inventive aspect. In another embodiment, the three-dimensional model of the at least one portion of the subject's heart and/or the torso model has been previously determined and stored in non-limiting examples of computer readable mediums such as electronic media, e.g. flash memories, optical media, e.g. CD-ROMs, magnetic media, e.g. hard disks or floppy disks, or any other storage media which is intended to be read by a computer. In this way, in step (i) and/or (iii) of the method, the three-dimensional model of the at least one portion of the subject's heart and/or the torso model can be loaded from any of the disclosed storage modalities.

In an embodiment, the body surface potentials (8) are measured and/or the position (5) of each sensor is determined as part of the method according to the second inventive aspect. In another embodiment, the body surface potentials (8) have been previously measured and/or the position (5) of each sensor has been previously determined and stored in non-limiting examples of computer readable mediums such as electronic media, e.g. flash memories, optical media, e.g. CD-ROMs, magnetic media, e.g. hard disks or floppy disks, or any other storage media which is intended to be read by a computer. In this way, in step (ii) of the method the body surface potentials (8) and/or the position (5) of each sensor can be loaded from any of the disclosed storage modalities.

In an embodiment, the location of the geometrical center (2) of the at least one portion of the subject's heart is determined as part of the method according to the second inventive aspect. In said embodiment, the method according to the second inventive aspect comprises the steps for determining the location of at least one portion of a subject's heart within the subject's torso according to the first inventive aspect. In said embodiment, steps (i) and (ii), and optionally steps (iii) and (iv), of the method according to the second inventive aspect are performed as part of the method according to the first inventive aspect. In another embodiment, the location of the geometrical center (2) of the at least one portion of the subject's heart has been previously determined and stored in non-limiting examples of computer readable mediums such as electronic media, e.g. flash memories, optical media, e.g. CD-ROMs, magnetic media, e.g. hard disks or floppy disks, or any other storage media which is intended to be read by a computer. In this way, in step (iv) of the method, the location of the geometrical center (2) of the at least one portion of the subject's heart can be loaded from any of the disclosed storage modalities.

### Cardiac morphology estimation

According to a third inventive aspect of the invention, a computer-implemented method for estimating the morphology of at least one portion of a subject's heart is provided, more specifically a method for providing a three-dimensional model of at least one portion of the heart (1).

In the context of the present invention, a three-dimensional model of the heart (1) or of a portion thereof is a convex hull comprising a plurality of vertices and a plurality of faces, each of said faces determined by at least three vertices and at least three edges connecting each pair of vertices. Particularly, each of said faces is determined by three vertices and three edges connecting each pair of vertices. In some particular embodiments, only a portion of the heart can be of interest, such as the upper or lower chambers of the heart. In such embodiments, the three-dimensional model of the at least one portion of the heart (1) is limited to a three-dimensional model of said portion, e.g. cardiac chambers, of interest. In other embodiments, the whole heart is of interest and the three-dimensional model of the at least one portion of the heart (1) is a three-dimensional model of the whole heart. Throughout this specification the "three-dimensional model of at least one portion of the subject's heart" will be also referred to as "cardiac model".

According to the third inventive aspect of the invention, the three-dimensional model of at least one portion of the subject's heart (1) is estimated by introducing deformations in a basal cardiac model, *G_{H}.* The method thus comprises providing a basal cardiac model, *G_{H},* that can be expressed as a function of a determined number of a determined number of deformation modes, *σ̅ₘ*, as: ${G}_{H} = f \left({\overline{σ}}_{1},{\overline{σ}}_{2},…,{\overline{σ}}_{m},…,{\overline{σ}}_{M}\right) , ∀ m ∈ \left[1, M\right] ,$ being M the total number of deformation modes, and *σ̅ₘ* the m-th deformation mode.

From said basal cardiac model *G_{H}*, the cardiac model of the subject, *G_{H}'*, is obtained by applying a weight, *αₘ*, to each deformation mode, so that: ${G}_{H} ′ = f \left({α}_{1}⋅{\overline{σ}}_{1},{α}_{2}⋅{\overline{σ}}_{2},…,{α}_{m}⋅{\overline{σ}}_{m},…,{α}_{M}⋅{\overline{σ}}_{M}\right) , ∀ m ∈ \left[1, M\right] ,$ being *αₘ* the weight assigned to the m-th deformation mode.

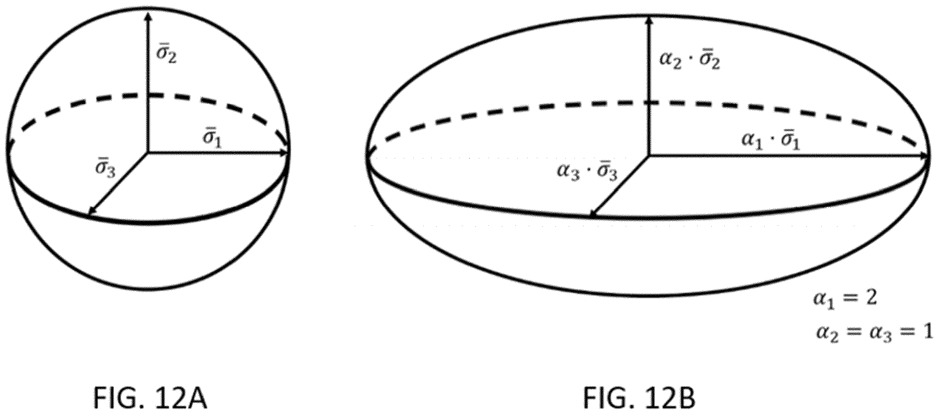

To serve as an example, if the first mode of deformation represents, e.g. the width of the cardiac model, the weights of the different deformation modes can be set as: ${α}_{1} = 2 ,$ ${α}_{2} = {α}_{3} = ⋯ = {α}_{m} = ⋯ = {α}_{M} , ∀ m ∈ \left[2, M\right] ,$ in order to obtain a cardiac model *G_{H}'* whose width is twice the width of the basal cardiac model *G_{H}.* An example of the mentioned deformation is displayed at FIGs. 12A-12B, wherein FIG. 12A shows the deformation modes of the basal cardiac model *G_{H}* and FIG. 12B shows the deformation modes of the modified cardiac model *G_{H}'.*

In an embodiment of the invention, the deformation modes are not associated with a particular spatial dimension or anatomical structure, but they are non-specific and can govern the morphological variation of the model in different spatial dimensions or anatomical structures.

In an embodiment, the basal cardiac model *G_{H}* is constructed as the average of a population of cardiac models. In an embodiment, the population of cardiac models comprises mathematical models of hearts (1) or of portions of hearts (1), each of them constructed to represent determined dimensions and morphologies in their anatomical structures. In an embodiment, the population of cardiac models comprises three-dimensional models generated from the segmentation of real cardiac geometries. In another embodiment the real cardiac geometries are obtained using an imaging system (9), such as computerized tomography, magnetic resonance, echography, angiography, and/or any other medical imaging modality suitable for the visualization of cardiac tissue. In yet another embodiment, the basal cardiac geometry is obtained by averaging a population of cardiac models comprising a combination of mathematical models and real cardiac geometries.

In an embodiment, the deformation modes of the basal cardiac model are computed by obtaining the principal components of the whole population of models, such as using a principal component analysis, and/or other non-limiting examples of algorithms such as independent component analysis, linear discriminant analysis, autoencoders, and/or non-limiting variations of the principal component analysis, such as kernel principal component analysis or graph-based principal component analysis.

The selection of the weights of each deformation mode to obtain a particular cardiac model can also be performed according to different embodiments. In an embodiment, said weights are obtained from a priori knowledge of the cardiac structure of the subject whose heart is to be represented, from at least one demographic feature of the subject and/or from at least one pathological feature of the subject. Said a priori knowledge can comprise information on the heart dimensions, such as the dimensions of the different cardiac chambers or any of their anatomic structures, or functional parameters, such as the degree of hypertrophy or the ejection fraction. Said heart dimensions and/or functional parameters can be obtained from non-limiting examples of medical imaging modalities such as computerized tomography, magnetic resonance, echography, angiography, or any other medical imaging modality suitable for the visualization of cardiac tissue.

In another embodiment, the weights of each deformation mode can be obtained by optimization of an inverse problem resolution. The inverse problem of cardiology is a mathematical problem which relates the electrical activity at each point of the cardiac surface (***U**_{H}*) with the body surface potentials (8) at each point of the torso surface (***U**_{T}*), by means of a transfer matrix **(*M*),** allowing to estimate ***U**_{H}* by departing from ***U**_{T}. **M*** can be estimated by determining the relationship between a three-dimensional model of the surface of the torso (*G_{T}*) and a three-dimensional model of the surface of the heart (*G_{H}*), by using non-limiting approaches such as the boundary elements method or the finite elements method. Therefore, ***U**_{H}* and ***U**_{T}* are related through ***M*** as: $M {U}_{H} = {U}_{T}$

However, the resolution of the inverse problem is ill-posed, this meaning that ***U**_{H}* cannot be obtained from ***U**_{T}* by simply inverting ***M*.** For this purpose, the resolution of the inverse problem requires the use of mathematical strategies, such as imposing constraints to the problem. One example is the use of Tikhonov's regularization, in which ***U**_{H}* are obtained by minimizing the following equation: ${\left‖M{U}_{H}-{U}_{T}\right‖}^{2} + λ {\left‖B{U}_{H}\right‖}^{2} ,$ where λ is a regularization parameter and ***B*** is a spatial regularization matrix. In the previous equation, the selection of λ can be based on several different techniques, one of which is the use of the so-called L-curve. The L-curve is a 2D representation in which the x and y axes represent log₁₀∥***MU**_{H}* - ***U**_{T}*∥² and log₁₀∥***BU**_{H}*∥², respectively, both as a function of λ. Afterwards, the optimal value for λ is obtained as that corresponding to the maximal curvature of the L-curve.

In a particular embodiment, a plurality of transfer matrixes, ***Mⁱ***, is constructed by departing from a plurality of cardiac models, ${G}_{H}^{i}$, obtained by the deformation of the basal cardiac model *G_{H}* using a plurality of combinations of the weights of its deformation modes. The obtention of the plurality of transfer matrixes, ***Mⁱ***, can be based on a variety of algorithms for the resolution of forward problems, such as the boundary elements method or the finite elements method. The inverse problem is then solved for each of the ***Mⁱ*** transfer matrixes, and the three-dimensional model of the at least one portion of the subject's heart, *G_{H}',* is selected from the plurality of estimated cardiac models, ${G}_{H}^{i}$, as the estimated cardiac model which satisfies a predefined condition. In an embodiment, the predefined condition is maximum curvature of the L-curve. According to this embodiment, for each of the ***Mⁱ*** transfer matrixes, a different L-curve is obtained, each of said L-curves having a different maximum curvature value. The selected combination of weights of the deformation modes employed to generate the target cardiac model (i.e. the three-dimensional model of the at least one portion of the subject's heart) is the one for which the maximum curvature of the L-curve is obtained.

In other embodiments, other methodologies for the resolution of the inverse problem or for the selection of the optimal regularization parameter can be employed. In these cases, a variety of cardiac models, ${G}_{H}^{i}$, can be obtained by the deformation of the basal cardiac model *G_{H}* using a plurality of combinations of the weights of its deformation modes. The inverse problem is then solved iteratively for each of said ${G}_{H}^{i}$, and the optimal combination of the weights of the deformation modes to be used is the one used to generate the model which satisfies a predefined condition, such as the minimization of a loss function.

In a particular embodiment, the basal cardiac model and their modes of deformation are stored in non-limiting examples of computer readable mediums such as electronic media, e.g. flash memories, optical media, e.g. CD-ROMs, magnetic media, e.g. hard disks or floppy disks, or any other storage media which is intended to be read by a computer. In this way, the basal cardiac model does not need to be computed each time, but it can be loaded from any of the disclosed storage modalities.

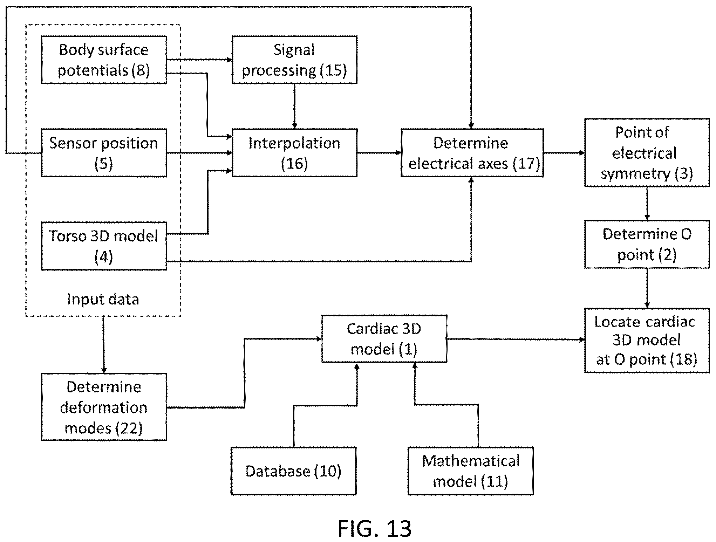

FIG. 13 shows an embodiment of the method according to a fourth inventive aspect of the invention, which provides a method for determining the location within the torso and the morphology of at least one portion of a subject's heart, the method comprising:
- determining the location of the at least one portion of the subject's heart within the torso according to the method of the first inventive aspect,
- determining the three-dimensional model of the at least one portion of the subject's heart according to the method of the third inventive aspect, and
- locating the three-dimensional model of the at least one portion of the subject's heart in the location of the geometrical center determined according to the method of the first inventive aspect.

In an embodiment, the method according to the fourth inventive aspect further comprises the following steps:
- solving the inverse problem of cardiology to generate an electroanatomical map, wherein the electrical activity of each area of the at least one portion of the subject's heart is identified;
- applying at least one electrocardiographic imaging (ECGI) analysis technique to the electroanatomical map; and
- based on the results of the ECGI analysis, determining at least one region of interest within the cardiac tissue.

In an embodiment, said region of interest depends on the particular application. Non-limiting examples of regions of interest are: the region of the cardiac tissue responsible for a cardiac arrhythmia, the most adequate location for the leads of an implantable pacemaker or defibrillator, or the region of the tissue presenting anatomical or functional anomalies, such as slow conduction velocity, fibrosis or dysplasia.

## Claims

1. A computer-implemented method for determining the location of at least one portion of a subject's heart (1) within the subject's torso, the method comprising the following steps:
(a) providing:
- a three-dimensional torso model (4) of the subject, the torso model (4) being defined by a plurality of vertices (12) and a plurality of faces (13), each of said faces (13) determined by at least three vertices (12) and at least three edges (14), each edge (14) connecting a pair of vertices (12),
- the position (5) of each sensor of a plurality of sensors placed on the subject's torso, and
- a signal corresponding to a body surface potential (8) measured by each sensor;
(b) interpolating (16) the body surface potential signal (8) in each vertex (12) of the torso model (4) based on the body surface potential signals (8) measured by the sensors;
(c) defining a plurality of electrical axes (17), each electrical axis (17) being an imaginary straight line connecting a vertex (12) with another vertex (12), the body surface potential signal (8) of which has the greatest morphological similarity but inverse polarity;
(d) determining the location of a point of electrical symmetry (3) in the torso model (4) based on the plurality of electrical axes (17); and
(e) determining the location of the geometrical center (2) of the at least one portion of the subject's heart in the torso model (4) based on the determined point of electrical symmetry (3).

2. The method according to claim 1, wherein step (c) comprises, for each vertex (12):
- determining the morphological similarity between the body surface potential signal (8) in said vertex (12) and the body surface potential signal (8) in each of the other vertices (12) of the torso model (4);
- comparing the determined morphological similarities; and
- defining the electrical axis (17) between said vertex (12) and the vertex (12) for which the morphological similarity is the highest and whose body surface potential signal (8) has inverse polarity.

3. The method according to any of the previous claims, wherein step (c) comprises determining, for each vertex (12):
- the correlation of the body surface potential signal (8) in said vertex (12) with the body surface potential signal (8) in each of the other vertices (12) and defining the electrical axis (17) between said vertex (12) and the vertex (12) having the greatest negative correlation; or
- the least squares value of the body surface potential signal (8) in said vertex (12) with the body surface potential signal (8) in each of the other vertices (12) and defining the electrical axis (17) between said vertex (12) and the vertex (12) having the lowest least squares value but inverse polarity; or
- the dynamic time warping deformation of the body surface potential signal (8) in said vertex (12) with the body surface potential signal (8) in each of the other vertices (12) and defining the electrical axis (17) between said vertex (12) and the vertex (12) having the lowest dynamic time warping deformation but inverse polarity; or
- the covariance of the body surface potential signal (8) in said vertex (12) with the body surface potential signal (8) in each of the other vertices (12) and defining the electrical axis (17) between said vertex (12) and the vertex (12) having the greatest negative covariance; or
- the vector distance of the body surface potential signal (8) in said vertex with the body surface potential signal (8) in each of the other vertices and defining the electrical axis between said vertex and the vertex having the lowest vector distance but inverse polarity.

4. The method according any of the previous claims, wherein in step (d) the location of a point of electrical symmetry (3) in the torso model (4) based on the plurality of electrical axes (17) is determined:
- as the intersection of the plurality of electrical axes (17); or
- within a region determined by the plurality of electrical axes (17).

5. The method according to any of the previous claims, wherein in step (e) the location of the geometrical center (2) of the at least one portion of the subject's heart in the torso model (4) is determined:
- by making coincident said geometrical center (2) with the point of electrical symmetry (3); or
- by applying an offset correction to the location of the point of electrical symmetry (3).

6. The method according to any of the previous claims, wherein the torso model (4) is obtained by any of the following options:
- automatic, semi-automatic, or manual segmentation of images obtained using a non-medical imaging system;
- automatic, semi-automatic or manual segmentation of images obtained using a medical imaging system;
- from a database of previously generated torso models; or
- generating a torso model from at least one mathematical model representing different subject characteristics;
and/or wherein the position (5) of each sensor on the torso of the subject is determined by any of the following options:
- automatic, semi-automatic or manual segmentation of images obtained using a non-medical imaging system;
- automatic, semi-automatic or manual segmentation of images obtained using a medical imaging system; or
- using an artificial intelligence-based approach for the identification of the sensor position based on the detection of readable codes, labels and/or drawings provided on the sensors.

7. The method according to any of the preceding claims, wherein step (d) comprises:
- computing a vector *d̅ᵢⱼ* for each pair of electrical axes *i* and *j*, being *d̅ᵢⱼ* a vector of magnitude *dᵢⱼ* and direction *d̂ᵢⱼ*, perpendicular to both electrical axes *i* and *j*; being *dᵢⱼ* the minimum distance between the electrical axes *i* and *j:* ${d}_{ij} = \left|\frac{\left({\vec{a}}_{j}-{\vec{a}}_{i}\right) ⋅ \left({\vec{b}}_{i}\times {\vec{b}}_{j}\right)}{\left|{\vec{b}}_{i}\times {\vec{b}}_{j}\right|}\right| ,$ wherein *a̅ᵢ* is a vector from the origin of coordinates to a point in electrical axis *i, a̅ⱼ* is a vector from the origin of coordinates to a point in electrical axis *j*, *b̅ᵢ* is a unit vector indicating the direction of electrical axis *i,* and *b̅ⱼ* is a unit vector indicating the direction of electrical axis *j*;
- computing, for each pair of electrical axes *i* and *j,* the midpoint of d̅ᵢ̅ⱼ̅; and
- defining the point of electrical symmetry (3) as the mean point of the plurality of midpoints of *d̅ᵢⱼ* computed: ${P}_{es} = \frac{1}{I ⋅ \left(I-1\right)} {\sum }_{i} {\sum }_{j \neq i} {μ}_{ij} ,$ wherein *Pₑₛ* is the point of electrical symmetry; *µᵢⱼ =* (*xᵢⱼ, yᵢⱼ, zᵢⱼ*) is the midpoint of *d̅ᵢⱼ* for a pair of electrical axes *i* and *j*; *i = 1, ... I*; *j* = 1, ... *I*; and *I* is the total number of electrical axes (17).

8. The method according to any of claims 1 to 6, further comprising, before step (d), defining a plurality of cylinders, each cylinder corresponding to an electrical axis (17) and having a longitudinal axis coaxial with the electrical axis (17) and a radius greater than 0;
wherein step (d) comprises:
- determining a total intersection volume (*V_{T}*), as the conjunction of the intersection volumes (*Vᵢⱼ*) that arise from the intersection of all the possible combinations of pairs of cylinders *i* and *j:* ${V}_{T} = {∪}_{i \neq j} {V}_{ij}$
- discretizing the total intersection volume (*V_{T}*) in voxels of a predetermined voxel size;
- quantifying the number of cylinders intersecting at each voxel of the discretized total intersection volume (*V_{T}*) and assigning the resulting value to said voxel, obtaining as a result a three-dimensional probability distribution function (21); and
- defining the point of electrical symmetry (3) as the center of the voxel for which the three-dimensional probability distribution function (21) is maximized.

9. The method according to any of the previous claims, further comprising:
- providing a three-dimensional model of the at least one portion of the subject's heart (1); and
- locating the model (1) of the at least one portion of the subject's heart in the determined location of the geometrical center (2) of the at least one portion of the subject's heart.

10. The method according to claim 9, wherein providing a three-dimensional model (1) of the at least one portion of the subject's heart comprises:
- providing a basal cardiac model, *G_{H}*, expressible as a function of a determined number of deformation modes, *σ̅ₘ*, as: ${G}_{H} = f \left({\overline{σ}}_{1},{\overline{σ}}_{2},…,{\overline{σ}}_{m},…,{\overline{σ}}_{M}\right) , ∀ m ∈ \left[1, M\right] ,$ being M the total number of deformation modes, and *σ̅ₘ* the m-th deformation mode;
- applying a weight, *αₘ*, to each deformation mode to obtain the three-dimensional model of at least one portion of the subject's heart, *G_{H}':* ${G}_{H} ′ = f \left({α}_{1}⋅{\overline{σ}}_{1},{α}_{2}⋅{\overline{σ}}_{2},…,{α}_{m}⋅{\overline{σ}}_{m},…,{α}_{M}⋅{\overline{σ}}_{M}\right) , ∀ m ∈ \left[1, M\right] ,$ being *αₘ* **the** weight assigned to the m-th deformation mode.

11. The method according to claim 10, wherein the basal cardiac model, *G_{H},* is constructed as an average of a population of cardiac models, the population of cardiac models comprising:
- mathematical models constructed to represent determined dimensions and/or morphologies of a heart or a portion thereof, and/or
- three-dimensional models generated from the segmentation of real cardiac geometries, the real cardiac geometries being particularly obtained using an imaging system (9).

12. The method according to any of claims 10 to 11, wherein the deformation modes of the basal cardiac model are computed by obtaining the principal components of the population of models.

13. The method according to any of claims 10 to 12, wherein the weights, *αₘ*, applied to each deformation mode to obtain the three-dimensional model (1) of at least one portion of the subject's heart are obtained from information representative of the cardiac structure of the subject, at least one demographic feature of the subject and/or at least one pathological feature of the subject.

14. The method according to any of claims 10 to 13, wherein:
- a plurality of estimated cardiac models, ${G}_{H}^{i}$, is obtained from the basal cardiac model, *G_{H},* by applying a plurality of combinations of weights of the deformation modes to the basal cardiac model, *G_{H}*;
- for each estimated cardiac model, ${G}_{H}^{i}$, a transfer matrix, ***Mⁱ*,** is estimated based on the relationship between the three-dimensional torso model (4) and the estimated cardiac model, ${G}_{H}^{i}$;
- for each estimated cardiac model, ${G}_{H}^{i}$, the inverse problem: ${M}^{i} {U}_{H}^{i} = {U}_{T}$ is solved,
***Mⁱ*** being the estimated transfer matrix, ${U}_{H}^{i}$ being the electrical activity at the cardiac surface of the estimated cardiac model, ${G}_{H}^{i}$*,* and *U_{T}* being the body surface potential signals (8) at the torso surface;
- the three-dimensional model of at least one portion of the subject's heart, *G_{H}',* is selected from the plurality of estimated cardiac models, ${G}_{H}^{i}$, as the estimated cardiac model which satisfies a predefined condition.

15. The method according to claim 14, wherein for each estimated cardiac model, ${G}_{H}^{i}$, the inverse problem is solved by minimizing the following equation: ${\left‖{M}^{i}{U}_{H}^{i}-{U}_{T}\right‖}^{2} + {λ}^{i} {\left‖{B}^{i}{U}_{H}^{i}\right‖}^{2} ,$ wherein *λⁱ* is a regularization parameter and *Bⁱ* is a spatial regularization matrix, wherein the Tikhonov regularization and L-curve method is used to select *λⁱ* for each estimated cardiac model, ${G}_{H}^{i}$*, λⁱ* being selected as the value corresponding to the maximal curvature of the L-curve; and wherein the three-dimensional model of at least one portion of the subject's heart, *G_{H}',* is selected as the estimated cardiac model, ${G}_{H}^{i}$*,* for which the maximum curvature of the L-curve is obtained.

16. A computer-implemented method for determining at least one region of interest within the cardiac tissue, the method comprising the following steps:
(i) providing a three-dimensional torso model (4) of the subject;
(ii) providing body surface potential signals (8) measured by a plurality of sensors placed on the subject's torso, and the position (5) of each sensor;
(iii) providing a three-dimensional model (1) of at least one portion of the subject's heart;
(iv) locating the three-dimensional model (1) of the at least one portion of the subject's heart at the location of the geometrical center (2) resulting from the method according to any of the previous claims;
(v) solving the inverse problem of cardiology to generate an electroanatomical map, wherein the electrical activity of each area of the at least one portion of the subject's heart is identified;
(vi) applying at least one electrocardiographic imaging analysis technique to the electroanatomical map; and
(vii) based on the results of the ECGI analysis, determining at least one region of interest within the cardiac tissue.

## Patentansprüche

1. Ein computerimplementiertes Verfahren zum Bestimmen der Lage mindestens eines Abschnitts des Herzens (1) einer Person innerhalb des Rumpfes der Person, wobei das Verfahren die folgenden Schritte umfasst:
(a) Bereitstellen:
- eines dreidimensionalen Rumpfmodells (4) der Person, wobei das Rumpfmodell (4) durch eine Vielzahl von Eckpunkten (12) und eine Vielzahl von Flächen (13) definiert ist, wobei jede der Flächen (13) durch mindestens drei Eckpunkte (12) und mindestens drei Kanten (14) bestimmt ist, wobei jede Kante (14) ein Paar von Eckpunkten (12) verbindet,
- der Position (5) jedes Sensors einer Vielzahl von Sensoren, die am Rumpf der Person angeordnet sind, und
- eines Signals, das einem von jedem Sensor gemessenen Körperoberflächenpotential (8) entspricht;
(b) Interpolieren (16) des Körperoberflächenpotentialsignals (8) an jedem Eckpunkt (12) des Rumpfmodells (4) auf Grundlage der von den Sensoren gemessenen Körperoberflächenpotentialsignale (8);
(c) Definieren einer Vielzahl von elektrischen Achsen (17), wobei jede elektrische Achse (17) eine gedachte gerade Linie ist, die einen Eckpunkt (12) mit einem anderen Eckpunkt (12) verbindet, dessen Körperoberflächenpotentialsignal (8) die größte morphologische Ähnlichkeit, jedoch umgekehrte Polarität aufweist;
(d) Bestimmen der Lage eines Punkts elektrischer Symmetrie (3) im Rumpfmodell (4) auf Grundlage der Vielzahl von elektrischen Achsen (17); und
(e) Bestimmen der Lage des geometrischen Mittelpunkts (2) des mindestens einen Abschnitts des Herzens der Person im Rumpfmodell (4) auf Grundlage des bestimmten Punkts elektrischer Symmetrie (3).

2. Das Verfahren nach Anspruch 1, wobei Schritt (c) für jeden Eckpunkt (12) umfasst:
- Bestimmen der morphologischen Ähnlichkeit zwischen dem Körperoberflächenpotentialsignal (8) an dem Eckpunkt (12) und dem Körperoberflächenpotentialsignal (8) an jedem der anderen Eckpunkte (12) des Rumpfmodells (4);
- Vergleichen der bestimmten morphologischen Ähnlichkeiten; und
- Definieren der elektrischen Achse (17) zwischen dem Eckpunkt (12) und dem Eckpunkt (12), für den die morphologische Ähnlichkeit am höchsten ist und dessen Körperoberflächenpotentialsignal (8) eine umgekehrte Polarität aufweist.

3. Das Verfahren nach einem der vorhergehenden Ansprüche, wobei Schritt (c) das Bestimmen, für jeden Eckpunkt (12), umfasst:
- der Korrelation des Körperoberflächenpotentialsignals (8) an dem Eckpunkt (12) mit dem Körperoberflächenpotentialsignal (8) an jedem der anderen Eckpunkte (12) und Definieren der elektrischen Achse (17) zwischen dem Eckpunkt (12) und dem Eckpunkt (12) mit der größten negativen Korrelation; oder
- des Kleinste-Quadrate-Werts des Körperoberflächenpotentialsignals (8) an dem Eckpunkt (12) mit dem Körperoberflächenpotentialsignal (8) an jedem der anderen Eckpunkte (12) und Definieren der elektrischen Achse (17) zwischen dem Eckpunkt (12) und dem Eckpunkt (12) mit dem niedrigsten Kleinste-Quadrate-Wert, jedoch umgekehrter Polarität; oder
- der Dynamic-Time-Warping-Verformung des Körperoberflächenpotentialsignals (8) an dem Eckpunkt (12) mit dem Körperoberflächenpotentialsignal (8) an jedem der anderen Eckpunkte (12) und Definieren der elektrischen Achse (17) zwischen dem Eckpunkt (12) und dem Eckpunkt (12) mit der geringsten Dynamic-Time-Warping-Verformung, jedoch umgekehrter Polarität; oder
- der Kovarianz des Körperoberflächenpotentialsignals (8) an dem Eckpunkt (12) mit dem Körperoberflächenpotentialsignal (8) an jedem der anderen Eckpunkte (12) und Definieren der elektrischen Achse (17) zwischen dem Eckpunkt (12) und dem Eckpunkt (12) mit der größten negativen Kovarianz; oder
- der Vektordistanz des Körperoberflächenpotentialsignals (8) an dem Eckpunkt zu dem Körperoberflächenpotentialsignal (8) an jedem der anderen Eckpunkte und Definieren der elektrischen Achse zwischen dem Eckpunkt und dem Eckpunkt mit der geringsten Vektordistanz, jedoch umgekehrter Polarität.

4. Das Verfahren nach einem der vorhergehenden Ansprüche, wobei in Schritt (d) die Lage eines Punkts elektrischer Symmetrie (3) im Rumpfmodell (4) auf Grundlage der Vielzahl von elektrischen Achsen (17) bestimmt wird:
- als der Schnittpunkt der Vielzahl von elektrischen Achsen (17); oder
- innerhalb eines durch die Vielzahl von elektrischen Achsen (17) bestimmten Bereichs.

5. Das Verfahren nach einem der vorhergehenden Ansprüche, wobei in Schritt (e) die Lage des geometrischen Mittelpunkts (2) des mindestens einen Abschnitts des Herzens der Person im Rumpfmodell (4) bestimmt wird:
- indem der geometrische Mittelpunkt (2) mit dem Punkt elektrischer Symmetrie (3) zur Deckung gebracht wird; oder
- indem eine Offset-Korrektur auf die Lage des Punkts elektrischer Symmetrie (3) angewendet wird.

6. Das Verfahren nach einem der vorhergehenden Ansprüche, wobei das Rumpfmodell (4) durch eine der folgenden Optionen erhalten wird:
- automatische, halbautomatische oder manuelle Segmentierung von Bildern, die unter Verwendung eines nichtmedizinischen Bildgebungssystems erhalten wurden;
- automatische, halbautomatische oder manuelle Segmentierung von Bildern, die unter Verwendung eines medizinischen Bildgebungssystems erhalten wurden;
- aus einer Datenbank zuvor erzeugter Rumpfmodelle; oder
- Erzeugen eines Rumpfmodells aus mindestens einem mathematischen Modell, das unterschiedliche Merkmale der Person darstellt;
und/oder wobei die Position (5) jedes Sensors am Rumpf der Person durch eine der folgenden Optionen bestimmt wird:
- automatische, halbautomatische oder manuelle Segmentierung von Bildern, die unter Verwendung eines nichtmedizinischen Bildgebungssystems erhalten wurden;
- automatische, halbautomatische oder manuelle Segmentierung von Bildern, die unter Verwendung eines medizinischen Bildgebungssystems erhalten wurden; oder
- Verwenden eines auf künstlicher Intelligenz basierenden Ansatzes zur Identifizierung der Sensorposition auf Grundlage der Erkennung von auf den Sensoren vorgesehenen lesbaren Codes, Kennzeichnungen und/oder Zeichnungen.

7. Das Verfahren nach einem der vorhergehenden Ansprüche, wobei Schritt (d) umfasst:
- Berechnen eines Vektors *d̅ᵢⱼ* für jedes Paar elektrischer Achsen *i* und *j,* wobei *d̅ᵢⱼ* ein Vektor mit dem Betrag *dᵢⱼ* und der Richtung *d̂ᵢⱼ* ist, der zu beiden elektrischen Achsen *i* und *j* senkrecht ist; wobei *dᵢⱼ* der minimale Abstand zwischen den elektrischen Achsen i und *j* ist: ${d}_{ij} = \left|\frac{\left({\vec{a}}_{j}-{\vec{a}}_{i}\right) ⋅ \left({\vec{b}}_{i}\times {\vec{b}}_{j}\right)}{\left|{\vec{b}}_{i}\times {\vec{b}}_{j}\right|}\right| ,$ wobei *a̅ᵢ* ein Vektor vom Koordinatenursprung zu einem Punkt auf der elektrischen Achse *i* ist, *a̅ⱼ* ein Vektor vom Koordinatenursprung zu einem Punkt auf der elektrischen Achse *j* ist, *b̅ᵢ* ein Einheitsvektor ist, der die Richtung der elektrischen Achse *i* angibt, und *b̅ⱼ* ein Einheitsvektor ist, der die Richtung der elektrischen Achse *j* angibt;
- Berechnen, für jedes Paar elektrischer Achsen *i* und *j*, des Mittelpunkts von *d̅ᵢⱼ*; und
- Definieren des Punkts elektrischer Symmetrie (3) als den mittleren Punkt der Vielzahl von berechneten Mittelpunkten von *d̅ᵢⱼ*: ${P}_{es} = \frac{1}{I ⋅ \left(I-1\right)} {\sum }_{i} {\sum }_{j \neq i} {μ}_{ij} ,$ wobei *Pₑₛ* der Punkt elektrischer Symmetrie ist; *µᵢⱼ =* (*xᵢⱼ, yᵢⱼ, zᵢⱼ*) der Mittelpunkt von *d̅ᵢⱼ* für ein Paar elektrischer Achsen *i* und *j* ist; *i* = *1, ...I; j* = 1*, ... I;* und *I* die Gesamtzahl der elektrischen Achsen (17) ist.

8. Das Verfahren nach einem der Ansprüche 1 bis 6, ferner umfassend, vor Schritt (d), das Definieren einer Vielzahl von Zylindern, wobei jeder Zylinder einer elektrischen Achse (17) entspricht und eine Längsachse aufweist, die koaxial zu der elektrischen Achse (17) ist, sowie einen Radius größer als 0;
wobei Schritt (d) umfasst:
- Bestimmen eines gesamten Schnittvolumens (*V_{T}*) als die Verknüpfung der Schnittvolumina (*Vᵢⱼ*), die sich aus dem Schnitt aller möglichen Kombinationen von Paaren von Zylindern *i* und *j* ergeben: ${V}_{T} = {∪}_{i \neq j} {V}_{ij}$
- Diskretisieren des gesamten Schnittvolumens (*V_{T}*) in Voxel einer vorbestimmten Voxelgröße;
- Quantifizieren der Anzahl von Zylindern, die sich an jedem Voxel des diskretisierten gesamten Schnittvolumens (*V_{T}*) schneiden, und Zuweisen des resultierenden Werts zu dem Voxel, wodurch als Ergebnis eine dreidimensionale Wahrscheinlichkeitsverteilungsfunktion (21) erhalten wird; und
- Definieren des Punkts elektrischer Symmetrie (3) als das Zentrum des Voxels, für das die dreidimensionale Wahrscheinlichkeitsverteilungsfunktion (21) maximiert ist.

9. Das Verfahren nach einem der vorhergehenden Ansprüche, ferner umfassend:
- Bereitstellen eines dreidimensionalen Modells (1) des mindestens einen Abschnitts des Herzens der Person; und
- Lokalisieren des Modells (1) des mindestens einen Abschnitts des Herzens der Person an der bestimmten Lage des geometrischen Mittelpunkts (2) des mindestens einen Abschnitts des Herzens der Person.

10. Das Verfahren nach Anspruch 9, wobei das Bereitstellen eines dreidimensionalen Modells (1) des mindestens einen Abschnitts des Herzens der Person umfasst:
- Bereitstellen eines kardialen Basismodells, *G_{H},* das als Funktion einer stimmten Anzahl von Deformationsmodi, *σ̅ₘ*, darstellbar ist, als: ${G}_{H} = f \left({\overline{σ}}_{1},{\overline{σ}}_{2},…,{\overline{σ}}_{m},…,{\overline{σ}}_{M}\right) , ∀ m ∈ \left[1, M\right] ,$ wobei M die Gesamtzahl der Deformationsmodi und *σ̅ₘ* der m-te Deformationsmodus ist;
- Anwenden einer Gewichtung, *αₘ,* auf jeden Deformationsmodus, um das dreidimensionale Modell mindestens eines Abschnitts des Herzens der Person, *G_{H}',* zu erhalten: ${G}_{H} ′ = f \left({α}_{1}⋅{\overline{σ}}_{1},{α}_{2}⋅{\overline{σ}}_{2},…,{α}_{m}⋅{\overline{σ}}_{m},…,{α}_{M}⋅{\overline{σ}}_{M}\right) , ∀ m ∈ \left[1, M\right] ,$ wobei *αₘ* die dem m-ten Deformationsmodus zugewiesene Gewichtung ist.

11. Das Verfahren nach Anspruch 10, wobei das kardiale Basismodell, *G_{H},* als Durchschnitt einer Population von Herzmodellen erstellt ist, wobei die Population von Herzmodellen umfasst:
- mathematische Modelle, die erstellt sind, um bestimmte Abmessungen und/oder Morphologien eines Herzens oder eines Abschnitts davon darzustellen, und/oder
- dreidimensionale Modelle, die aus der Segmentierung realer Herzgeometrien erzeugt sind, wobei die realen Herzgeometrien insbesondere unter Verwendung eines Bildgebungssystems (9) erhalten werden.

12. Das Verfahren nach einem der Ansprüche 10 bis 11, wobei die Deformationsmodi des kardialen Basismodells durch Ermitteln der Hauptkomponenten der Population von Modellen berechnet werden.

13. Das Verfahren nach einem der Ansprüche 10 bis 12, wobei die Gewichtungen, *αₘ*, die auf jeden Deformationsmodus angewendet werden, um das dreidimensionale Modell (1) mindestens eines Abschnitts des Herzens der Person zu erhalten, aus Informationen erhalten werden, die für die Herzstruktur der Person, mindestens ein demografisches Merkmal der Person und/oder mindestens ein pathologisches Merkmal der Person repräsentativ sind.

14. Das Verfahren nach einem der Ansprüche 10 bis 13, wobei:
- eine Vielzahl geschätzter kardialer Modelle, ${G}_{H}^{i}$, aus dem kardialen Basismodell, *G_{H}*, erhalten wird, indem eine Vielzahl von Kombinationen von Gewichtungen der Deformationsmodi auf das kardiale Basismodell, *G_{H}*, angewendet wird;
- für jedes geschätzte kardiale Modell, ${G}_{H}^{i}$, eine Übertragungsmatrix, ***M**ⁱ*, auf Grundlage der Beziehung zwischen dem dreidimensionalen Rumpfmodell (4) und dem geschätzten kardialen Modell, ${G}_{H}^{i}$, geschätzt wird;
- für jedes geschätzte kardiale Modell, ${G}_{H}^{i}$; , das inverse Problem: ${M}^{i} {U}_{H}^{i} = {U}_{T}$ gelöst wird,
wobei *Mⁱ* die geschätzte Übertragungsmatrix ist, ${U}_{H}^{i}$ die elektrische Aktivität an der Herzoberfläche des geschätzten kardialen Modells, ${G}_{H}^{i}$, ist, und *U_{T}* die Körperoberflächenpotentialsignale (8) an der Rumpfoberfläche sind;
- das dreidimensionale Modell mindestens eines Abschnitts des Herzens der Person, *G_{H}*', aus der Vielzahl geschätzter kardialer Modelle, ${G}_{H}^{i}$, als das geschätzte kardiale Modell ausgewählt wird, das eine vorgegebene Bedingung erfüllt.

15. Das Verfahren nach Anspruch 14, wobei für jedes geschätzte kardiale Modell, ${G}_{H}^{i}$, das inverse Problem durch Minimieren der folgenden Gleichung gelöst wird: ${\left‖{M}^{i}{U}_{H}^{i}-{U}_{T}\right‖}^{2} + {λ}^{i} {\left‖{B}^{i}{U}_{H}^{i}\right‖}^{2} ,$ wobei *λⁱ* ein Regularisierungsparameter und *Bⁱ* eine räumliche Regularisierungsmatrix ist, wobei die Tikhonov-Regularisierung und die L-Kurven-Methode verwendet werden, um *λⁱ* für jedes geschätzte kardiale Modell, ${G}_{H}^{i}$, auszuwählen, wobei *λⁱ* als der Wert ausgewählt wird, der der maximalen Krümmung der L-Kurve entspricht; und wobei das dreidimensionale Modell mindestens eines Abschnitts des Herzens der Person, *G_{H}*', als das geschätzte kardiale Modell, ${G}_{H}^{i}$, ausgewählt wird, für das die maximale Krümmung der L-Kurve erhalten wird.

16. Ein computerimplementiertes Verfahren zum Bestimmen mindestens einer Region von Interesse innerhalb des Herzgewebes, wobei das Verfahren die folgenden Schritte umfasst:
(i) Bereitstellen eines dreidimensionalen Rumpfmodells (4) der Person;
(ii) Bereitstellen von Körperoberflächenpotentialsignalen (8), die von einer Vielzahl von am Rumpf der Person angeordneten Sensoren gemessen werden, und der Position (5) jedes Sensors;
(iii) Bereitstellen eines dreidimensionalen Modells (1) mindestens eines Abschnitts des Herzens der Person;
(iv) Lokalisieren des dreidimensionalen Modells (1) des mindestens einen Abschnitts des Herzens der Person an der Lage des geometrischen Mittelpunkts (2), die sich aus dem Verfahren nach einem der vorhergehenden Ansprüche ergibt;
(v) Lösen des inversen Problems der Kardiologie, um eine elektroanatomische Karte zu erzeugen, wobei die elektrische Aktivität jedes Bereichs des mindestens einen Abschnitts des Herzens der Person identifiziert wird;
(vi) Anwenden mindestens einer Analysetechnik der elektrokardiographischen Bildgebung auf die elektroanatomische Karte; und
(vii) Bestimmen mindestens einer Region von Interesse innerhalb des Herzgewebes auf Grundlage der Ergebnisse der ECGI-Analyse.

## Revendications

1. Un procédé mis en œuvre par ordinateur pour déterminer l'emplacement d'au moins une partie du cœur (1) d'un sujet à l'intérieur du torse du sujet, le procédé comprenant les étapes suivantes :
(a) fournir :
- un modèle tridimensionnel du torse (4) du sujet, le modèle du torse (4) étant défini par une pluralité de sommets (12) et une pluralité de faces (13), chacune desdites faces (13) étant déterminée par au moins trois sommets (12) et au moins trois arêtes (14), chaque arête (14) reliant une paire de sommets (12) ;
- la position (5) de chaque capteur parmi une pluralité de capteurs disposés sur le torse du sujet ; et
- un signal correspondant à un potentiel de surface corporelle (8), mesuré par chaque capteur ;
(b) interpoler (16) le signal de potentiel de surface corporelle (8) en chaque sommet (12) du modèle du torse (4), sur la base des signaux de potentiel de surface corporelle (8) mesurés par les capteurs ;
(c) définir une pluralité d'axes électriques (17), chaque axe électrique (17) étant une droite imaginaire reliant un sommet (12) à un autre sommet (12), le signal de potentiel de surface corporelle (8) de celui-ci présentant la plus grande similarité morphologique, mais une polarité inverse ;
(d) déterminer l'emplacement d'un point de symétrie électrique (3) dans le modèle du torse (4) sur la base de la pluralité d'axes électriques (17) ; et
(e) déterminer l'emplacement du centre géométrique (2) de ladite au moins une partie du cœur du sujet dans le modèle du torse (4) sur la base du point de symétrie électrique (3) déterminé.

2. Le procédé selon la revendication 1, dans lequel l'étape (c) comprend, pour chaque sommet (12) :
- la détermination de la similarité morphologique entre le signal de potentiel de surface corporelle (8) dans ledit sommet (12) et le signal de potentiel de surface corporelle (8) en chacun des autres sommets (12) du modèle du torse (4) ;
- la comparaison des similarités morphologiques déterminées ; et
- la définition de l'axe électrique (17) entre ledit sommet (12) et le sommet (12) pour lequel la similarité morphologique est la plus élevée et dont le signal de potentiel de surface corporelle (8) présente une polarité inverse.

3. Le procédé selon l'une quelconque des revendications précédentes, dans lequel l'étape (c) comprend la détermination, pour chaque sommet (12) :
- de la corrélation du signal de potentiel de surface corporelle (8) dans ledit sommet (12) avec le signal de potentiel de surface corporelle (8) en chacun des autres sommets (12) et la définition de l'axe électrique (17) entre ledit sommet (12) et le sommet (12) ayant la plus grande corrélation négative ; ou
- de la valeur des moindres carrés du signal de potentiel de surface corporelle (8) dans ledit sommet (12) avec le signal de potentiel de surface corporelle (8) en chacun des autres sommets (12) et la définition de l'axe électrique (17) entre ledit sommet (12) et le sommet (12) ayant la plus faible valeur des moindres carrés mais une polarité inverse ; ou
- de la déformation temporelle dynamique du signal de potentiel de surface corporelle (8) dans ledit sommet (12) avec le signal de potentiel de surface corporelle (8) en chacun des autres sommets (12) et la définition de l'axe électrique (17) entre ledit sommet (12) et le sommet (12) ayant la plus faible déformation par déformation temporelle dynamique mais une polarité inverse ; ou
- de la covariance du signal de potentiel de surface corporelle (8) dans ledit sommet (12) avec le signal de potentiel de surface corporelle (8) en chacun des autres sommets (12) et la définition de l'axe électrique (17) entre ledit sommet (12) et le sommet (12) ayant la plus grande covariance négative ; ou
- de la distance vectorielle du signal de potentiel de surface corporelle (8) dans ledit sommet avec le signal de potentiel de surface corporelle (8) en chacun des autres sommets et la définition de l'axe électrique entre ledit sommet et le sommet ayant la plus faible distance vectorielle mais une polarité inverse.

4. Le procédé selon l'une quelconque des revendications précédentes, dans lequel, à l'étape (d), l'emplacement d'un point de symétrie électrique (3) dans le modèle du torse (4) sur la base de la pluralité d'axes électriques (17) est déterminé :
- comme étant l'intersection de la pluralité d'axes électriques (17) ; ou
- à l'intérieur d'une région déterminée par la pluralité d'axes électriques (17).

5. Le procédé selon l'une quelconque des revendications précédentes, dans lequel, à l'étape (e), l'emplacement du centre géométrique (2) de ladite au moins une partie du cœur du sujet dans le modèle du torse (4) est déterminé :
- en faisant coïncider ledit centre géométrique (2) avec le point de symétrie électrique (3) ; ou
- en appliquant une correction de décalage à l'emplacement du point de symétrie électrique (3).

6. Le procédé selon l'une quelconque des revendications précédentes, dans lequel le modèle du torse (4) est obtenu par l'une quelconque des options suivantes :
- segmentation automatique, semi-automatique ou manuelle d'images obtenues à l'aide d'un système d'imagerie non médicale ;
- segmentation automatique, semi-automatique ou manuelle d'images obtenues à l'aide d'un système d'imagerie médicale ;
- à partir d'une base de données de modèles de torse précédemment générés ; ou
- génération d'un modèle de torse à partir d'au moins un modèle mathématique représentant différentes caractéristiques du sujet ;
et/ou dans lequel la position (5) de chaque capteur sur le torse du sujet est déterminée par l'une quelconque des options suivantes :
- segmentation automatique, semi-automatique ou manuelle d'images obtenues à l'aide d'un système d'imagerie non médicale ;
- segmentation automatique, semi-automatique ou manuelle d'images obtenues à l'aide d'un système d'imagerie médicale ; ou
- utilisation d'une approche fondée sur l'intelligence artificielle pour l'identification de la position du capteur sur la base de la détection de codes lisibles, d'étiquettes et/ou de marquages fournis sur les capteurs.

7. Le procédé selon l'une quelconque des revendications précédentes, dans lequel l'étape (d) comprend :
- le calcul d'un vecteur *d̅ᵢⱼ* pour chaque paire d'axes électriques i et *j, d̅ᵢⱼ* étant un vecteur de magnitude *dᵢⱼ* et de direction *d̂ᵢⱼ,* perpendiculaire à la fois aux axes électriques *i* et *j; dᵢⱼ* étant la distance minimale entre les axes électriques i et *j :* ${d}_{ij} = \left|\frac{\left({\vec{a}}_{j}-{\vec{a}}_{i}\right) ⋅ \left({\vec{b}}_{i}\times {\vec{b}}_{j}\right)}{\left|{\vec{b}}_{i}\times {\vec{b}}_{j}\right|}\right| ,$ dans lequel *a̅ᵢ* est un vecteur de l'origine des coordonnées à un point de l'axe électrique *i, a̅ⱼ* est un vecteur de l'origine des coordonnées à un point de l'axe électrique *j, b̅ᵢ* est un vecteur unitaire indiquant la direction de l'axe électrique i, et *b̅ⱼ* est un vecteur unitaire indiquant la direction de l'axe électrique *j*;
- le calcul, pour chaque paire d'axes électriques i et *j,* du point médian de *d̅ᵢⱼ;* et
- la définition du point de symétrie électrique (3) comme le point moyen de la pluralité de points médians de *d̅ᵢⱼ* calculés : ${P}_{es} = \frac{1}{I ⋅ \left(I-1\right)} {\sum }_{i} {\sum }_{j \neq i} {μ}_{ij} ,$ **dans lequel** *Pₑₛ* est le point de symétrie électrique ; *µᵢⱼ =* (*xᵢⱼ, yᵢⱼ, zᵢⱼ*) est le point médian de *d̅ᵢⱼ* pour une paire d'axes électriques i et *j ; i* = 1, *...I* ; *j =* 1, *...I* ; et *I* est le nombre total d'axes électriques (17).

8. Le procédé selon l'une quelconque des revendications 1 à 6, comprenant en outre, avant l'étape (d), la définition d'une pluralité de cylindres, chaque cylindre correspondant à un axe électrique (17) et ayant un axe longitudinal coaxial avec l'axe électrique (17) et un rayon supérieur à 0 ;
dans lequel l'étape (d) comprend :
- la détermination d'un volume d'intersection total (*V_{T}*)*,* comme étant la conjonction des volumes d'intersection (*Vᵢⱼ*) qui résultent de l'intersection de toutes les combinaisons possibles de paires de cylindres *i* et *j* : ${V}_{T} = {∪}_{i \neq j} {V}_{ij}$
- la discrétisation du volume d'intersection total (*V_{T}*) en voxels d'une taille de voxel prédéterminée ;
- la quantification du nombre de cylindres se croisant en chaque voxel du volume d'intersection total discrétisé (*V_{T}*) et l'attribution de la valeur résultante audit voxel, obtenant comme résultat une fonction de distribution de probabilité tridimensionnelle (21) ; et
- la définition du point de symétrie électrique (3) comme étant le centre du voxel pour lequel la fonction de distribution de probabilité tridimensionnelle (21) est maximisée.

9. Le procédé selon l'une quelconque des revendications précédentes, comprenant en outre :
- la fourniture d'un modèle tridimensionnel de ladite au moins une partie du cœur (1) du sujet ; et
- le positionnement du modèle (1) de ladite au moins une partie du cœur du sujet à l'emplacement déterminé du centre géométrique (2) de ladite au moins une partie du cœur du sujet.

10. Le procédé selon la revendication 9, dans lequel la fourniture d'un modèle tridimensionnel (1) de ladite au moins une partie du cœur du sujet comprend :
- la fourniture d'un modèle cardiaque de base, *G_{H},* exprimable en fonction d'un nombre déterminé de modes de déformation, *σ̅ₘ*, comme suit : ${G}_{H} = f \left({\overline{σ}}_{1},{\overline{σ}}_{2},…,{\overline{σ}}_{m},…,{\overline{σ}}_{M}\right) , ∀ m ∈ \left[1, M\right] ,$ M étant le nombre total de modes de déformation, et *σ̅ₘ* le m-ième mode de déformation ;
- l'application d'un poids, *αₘ*, à chaque mode de déformation pour obtenir le modèle tridimensionnel de ladite au moins une partie du cœur du sujet, *G_{H}' :* ${G}_{H} ′ = f \left({α}_{1}⋅{\overline{σ}}_{1},{α}_{2}⋅{\overline{σ}}_{2},…,{α}_{m}⋅{\overline{σ}}_{m},…,{α}_{M}⋅{\overline{σ}}_{M}\right) , ∀ m ∈ \left[1, M\right] ,$ *αₘ* étant le poids attribué au m-ième mode de déformation.

11. Le procédé selon la revendication 10, dans lequel le modèle cardiaque de base, *G_{H},* est construit comme une moyenne d'une population de modèles cardiaques, la population de modèles cardiaques comprenant :
- des modèles mathématiques construits pour représenter des dimensions et/ou morphologies déterminées d'un cœur ou d'une partie de celui-ci, et/ou
- des modèles tridimensionnels générés à partir de la segmentation de géométries cardiaques réelles, les géométries cardiaques réelles étant particulièrement obtenues à l'aide d'un système d'imagerie (9).

12. Le procédé selon l'une quelconque des revendications 10 à 11, dans lequel les modes de déformation du modèle cardiaque de base sont calculés en obtenant les composantes principales de la population de modèles.

13. Le procédé selon l'une quelconque des revendications 10 à 12, dans lequel les poids, *αₘ*, appliqués à chaque mode de déformation pour obtenir le modèle tridimensionnel (1) d'au moins une partie du cœur du sujet sont obtenus à partir d'informations représentatives de la structure cardiaque du sujet, d'au moins une caractéristique démographique du sujet et/ou d'au moins une caractéristique pathologique du sujet.

14. Le procédé selon l'une quelconque des revendications 10 à 13, dans lequel :
- une pluralité de modèles cardiaques estimés, ${G}_{H}^{i}$*,* est obtenue à partir du modèle cardiaque de base, *G_{H},* en appliquant une pluralité de combinaisons de poids des modes de déformation au modèle cardiaque de base, *G_{H} ;*
- pour chaque modèle cardiaque estimé, ${G}_{H}^{i}$*,* une matrice de transfert, *Mⁱ*, est estimée sur la base de la relation entre le modèle de torse tridimensionnel (4) et le modèle cardiaque estimé, ${G}_{H}^{i}$*;*
- pour chaque modèle cardiaque estimé, ${G}_{H}^{i}$*,* le problème inverse : ${M}^{i} {U}_{H}^{i} = {U}_{T}$ est résolu,
*Mⁱ* étant la matrice de transfert estimée, ${U}_{H}^{i}$*,* étant l'activité électrique à la surface cardiaque du modèle cardiaque estimé, ${G}_{H}^{i}$*,* et ***U**_{T}* étant les signaux de potentiel de surface corporelle (8) à la surface du torse ;
- le modèle tridimensionnel d'au moins une partie du cœur du sujet, *G_{H}',* est sélectionné parmi la pluralité de modèles cardiaques estimés, ${G}_{H}^{i}$*,* comme étant le modèle cardiaque estimé qui satisfait une condition prédéfinie.

15. Le procédé selon la revendication 14, dans lequel pour chaque modèle cardiaque estimé, ${G}_{H}^{i}$*,* le problème inverse est résolu en minimisant l'équation suivante : ${\left‖{M}^{i}{U}_{H}^{i}-{U}_{T}\right‖}^{2} + {λ}^{i} {\left‖{B}^{i}{U}_{H}^{i}\right‖}^{2} ,$ dans laquelle *λⁱ* est un paramètre de régularisation et ***Bⁱ*** est une matrice de régularisation spatiale, dans laquelle la régularisation de Tikhonov et la méthode de la courbe en L sont utilisées pour sélectionner *λⁱ* pour chaque modèle cardiaque estimé, ${G}_{H}^{i}$*, λⁱ* étant sélectionné comme la valeur correspondant à la courbure maximale de la courbe en L ; et dans laquelle le modèle tridimensionnel d'au moins une partie du cœur du sujet, *G_{H}',* est sélectionné comme le modèle cardiaque estimé, ${G}_{H}^{i}$*,* pour lequel la courbure maximale de la courbe en L est obtenue.

16. Un procédé mis en œuvre par ordinateur pour déterminer au moins une région d'intérêt dans le tissu cardiaque, le procédé comprenant les étapes suivantes :
(i) fournir un modèle tridimensionnel de torse (4) du sujet ;
(ii) fournir des signaux de potentiel de surface corporelle (8) mesurés par une pluralité de capteurs placés sur le torse du sujet, et la position (5) de chaque capteur ;
(iii) fournir un modèle tridimensionnel (1) d'au moins une partie du cœur du sujet ;
(iv) positionner le modèle tridimensionnel (1) de ladite au moins une partie du cœur du sujet à l'emplacement du centre géométrique (2) résultant du procédé selon l'une quelconque des revendications précédentes ;
(v) résoudre le problème inverse de cardiologie pour générer une carte électroanatomique, dans laquelle l'activité électrique de chaque zone de ladite au moins une partie du cœur du sujet est identifiée ;
(vi) appliquer au moins une technique d'analyse d'imagerie électrocardiographique à la carte électroanatomique ; et
(vii) sur la base des résultats de l'analyse ECGI, déterminer au moins une région d'intérêt dans le tissu cardiaque.
